# EUROPEAN PATENT SPECIFICATION

(11) **EP 3 972 537 B1**
(45) Date of publication and mention of the grant of the patent: **15.11.2023**
(21) Application number: 20729199.8
(22) Date of filing: 19.05.2020
(51) Int. Cl.: A61F 2/58, A61F 2/68, A61F 2/70

(54) **ACTUATION SYSTEMS FOR PROSTHETIC DIGITS**
BETÄTIGUNGSSYSTEME FÜR FINGERPROTHESEN
SYSTÈMES D'ACTIONNEMENT POUR LES PROTHÈSES DE DOIGTS

(30) Priority: 21.05.2019 US 201962850675 P
(43) Date of publication of application: 30.03.2022
(73) Proprietor: Touch Bionics Limited, Livingston Lothian EH54 7EG (GB)
(72) Inventor: BENNING, Matthew James, Livingston Lothian EH54 7EG (GB)
(74) Representative: KIPA AB
(86) International application number: PCT/IB2020/054748
(87) International publication number: WO 2020/234777

(56) References cited:
- EP-A1- 2 612 619
- EP-A1- 2 719 361
- WO-A1-2013/038143
- WO-A1-2014/027897
- US-A- 4 084 267

## Description

The present application claims priority to U.S. Provisional Patent Application No. 62/850,675, titled "Actuation Systems for Prosthetic Digits" and filed on May 21, 2019, which is hereby referred to.

### BACKGROUND

### Field

Features for prosthetics are described, in particular prosthetic digits.

### Description of the Related Art

Prosthetic digits are useful for amputees missing natural fingers. Existing solutions to prosthetic digits do not sufficiently mimic natural fingers and so functionality is not fully restored. Improvements to prosthetic digits are therefore desirable.

EP 2 612 619 A1 discloses an artificial hand component comprising a drive motor and at least two digits coupled to be driven by the drive motor. The said at least two digits are coupled to be driven by the drive motor via the intermediary of differential gearing.

WO 2014/027897 A1 discloses a gripping device, such as an automated hand, comprising a plurality of metacarpal members, each metacarpal member having a first end attached to a support frame via a universal joint and having a second end attached to a gripping member, wherein the metacarpal members are able to pivot upwardly, downwardly and laterally. Where adjacent metacarpal members together form a palm of the hand, the ability of the metacarpal members to move up, down and laterally helps the palm to grip an object and also helps the palm to withstand at least some forces from above, below and from the side.

### SUMMARY

The embodiments disclosed herein each have several aspects no single one of which is solely responsible for the disclosure's desirable attributes. Without limiting the scope of this disclosure, its more prominent features will now be briefly discussed. After considering this discussion, and particularly after reading the section entitled "Detailed Description," one will understand how the features of the embodiments described herein provide advantages over existing systems, devices and methods for prosthetic digits.

The invention concerns an actuator as defined in claim 1 and a prosthetic digit comprising such an actuator as defined in claim 5. The following disclosure describes non-limiting examples of some actuators and prosthetic digits.

Features for prosthetic digits are described. The digits mimic natural fingers by having three articulating segments, including a proximal, middle and distal segment. The segments are articulated by an actuator and mechanical links configured to cause rotation of the segments. The digit may have multiple degrees of freedom. A single actuator may be used for a single digit. A tendon may be used in some versions. The rotated digit may provide articulation that mimics a natural finger and thus fully surrounds a variety of shapes and sizes of objects to provide and restore enhanced gripping functionality to amputees. The digit provides space, weight and power savings due to the need for only a single actuator. A spring-biased worm wheel transmission provides a manual mode for rotation of the digit and prevents damage due to rotation induced by external forces acting on the digit. Actuation systems for the prosthetic digits may include the compact actuator that expands linearly to rotate the digit. Each digit may have its own actuator, which may be housed in the digit and/or the palm. A motor may rotate a leadscrew. The leadscrew may engage and move axially a housing or other member. Axial movement of the housing or member causes the proximal digit segment to pivot and thus the digit to articulate. In some embodiments, the leadscrew may rotate a wheel to actuate a tendon. An actuation tendon may cause a closing rotation of the digit segments, and a return tendon may cause an opening rotation.

In one aspect, a prosthetic digit is described. The prosthetic digit comprises a mount, a proximal segment, a middle segment, a distal segment, a proximal link, a distal link, and an actuator. The mount is configured to attach to a hand. The proximal segment is rotatably attached to the mount at a first pivot, and the middle segment is rotatably attached to the proximal and distal segments. The proximal link is rotatably attached to the mount and rotatably attached to the middle segment at a second pivot. The distal link is rotatably attached to the proximal link and rotatably attached to the distal segment at a third pivot. The actuator is coupled with the mount and the proximal segment, and the actuator is configured to cause the proximal segment to rotate about the first pivot, where rotation of the proximal segment about the first pivot causes the middle and distal segments to rotate.

In another aspect, a prosthetic digit is described. The prosthetic digit comprises a mount, a plurality of articulating segments comprising a proximal articulating segment, and an actuator. The mount is configured to attach to a hand. The proximal segment is rotatably attached to the mount at a first pivot and is rotatably attached to the actuator at a first j oint. The first j oint is located offset from the first pivot, such that linear actuation output by the actuator imposes a force at the first joint to cause the proximal segment to rotate about the first pivot.

In another aspect, a prosthetic hand is described that includes the prosthetic digit.

In another aspect, a prosthetic digit is described that comprises a mount, a proximal segment, a middle segment, a distal segment, a proximal expandable link, and an actuator. The mount is configured to attach to a hand. The proximal segment is rotatably attached to the mount, and the middle segment is rotatably attached to the proximal and distal segments. The proximal expandable link is rotatably coupled with the mount and configured to expand linearly such that the middle and distal segments can rotate independently of rotation of the proximal segment. The actuator is in mechanical communication with the middle and distal segments and configured to cause the middle and distal segments to rotate. In some embodiments, the actuator is in mechanical communication with the proximal segment via a tendon.

In another aspect, an actuator for a prosthetic digit is described that comprises a gearbox, a motor, a shaft, a leadscrew, and a housing. The motor is in mechanical communication with the gearbox. The shaft extends axially and distally from the gearbox. The leadscrew is coupled to the shaft and has an external thread. The motor is configured to cause the leadscrew to rotate in a first rotational direction. The housing is configured to couple with a prosthetic digit. The housing has an internal thread configured to engage the external thread of the leadscrew. Rotation of the leadscrew causes the housing to translate axially relative to the leadscrew to thereby cause the prosthetic digit to rotate.

In another aspect, a prosthetic digit is described that comprises an actuator having a mount, a motor, a leadscrew, and a housing. The mount is configured to attach to a hand. The motor is supported by the mount. The leadscrew is coupled with the motor and has an external thread. The motor is configured to cause the leadscrew to rotate about a first axis. The housing extends along the first axis and is configured to couple with a proximal end of a prosthetic digit. The housing defines an internal cavity having an internal thread that is engaged with the external thread of the leadscrew. Rotation of the leadscrew causes the housing to translate along the first axis to thereby cause the prosthetic digit to rotate.

In another aspect, a prosthetic digit is described that comprises a base, a proximal segment, a middle segment, a distal segment, an actuator, a wheel, a tendon, a tendon guide, and an expandable link. The base is configured to attach to a prosthetic hand. The proximal segment is rotatably attached to the base. The middle segment is rotatably attached to the proximal and distal segments. The actuator is coupled with the base. The wheel is in mechanical communication with the actuator. The actuator is configured to rotate the wheel about a first axis. The tendon is coupled with the wheel and extending distally therefrom. The tendon guide is coupled with the prosthetic digit and the tendon extends along the tendon guide. The expandable link extends from a proximal end to a distal end. The proximal end is rotatably attached to the base about the first axis and the second end is rotatably attached with the middle segment. The actuator is configured to rotate the wheel in a first rotational direction to thereby pull the tendon proximally to cause the distal segment to rotate relative to the middle segment in a first rotational direction. The distal end of the expandable link is configured to extend distally relative to the proximal end of the expandable link to thereby allow the middle and distal segments to rotate independently of rotation of the proximal segment.

In some examples, the prosthetic digit further comprises a distal link rotatably coupled with the proximal expandable link and with the distal segment.

In some examples, the proximal expandable link comprises a proximal portion, a distal portion, and a spring, where the proximal portion is in mechanical communication with the distal portion via the spring.

In another aspect, a prosthetic digit is described that comprises a mount, a plurality of articulating segments, and an actuator. The mount is configured to attach to a hand. The plurality of articulating segments comprise a proximal articulating segment. The proximal segment is rotatably attached to the mount at a first pivot, the proximal segment is rotatably attached to the actuator at a first joint, and the first joint is located offset from the first pivot, such that linear actuation output by the actuator imposes a force at the first joint to cause the proximal segment to rotate about the first pivot.

### BRIEF DESCRIPTION OF THE DRAWINGS

FIGS. 1A-1B are side and front views, respectively, of a lower arm stump having embodiments of prosthetic digits attached thereto, which prosthetic digits may be any of the prosthetic digits described herein.
FIGS. 2A-2B are back and front views, respectively, of a prosthetic hand incorporating embodiments of prosthetic digits, which prosthetic digits may be any of the prosthetic digits described herein.
FIGS. 3A-3D are various views of an embodiment of a prosthetic digit, having articulating proximal, middle and distal segments and mechanically-connected rigid links, that may be used with the lower arm stump of FIGS. 1A-1B or prosthetic hand of FIGS. 2A-2B.
FIG. 3E is a partially exploded perspective view of the prosthetic digit of FIGS. 3A-3D.
FIGS. 3F-3H are sequential views of the prosthetic digit of FIGS. 3A-3D shown in various rotated configurations where the middle and distal segments rotate as the proximal segment rotates due to interaction of the links.
FIGS. 4A-4D are various views of another embodiment of a prosthetic digit, having articulating proximal, middle and distal segments and an expandable proximal link, that may be used with the lower arm stump of FIGS. 1A-1B or prosthetic hand of FIGS. 2A-2B.
FIGS. 5A-5E are various views of the expandable link used in the prosthetic digit of FIGS. 4A-4D.
FIGS. 6A-6D are sequential views of the prosthetic digit of FIGS. 4A-4D shown in various rotated configurations where the middle and distal segments rotate as the proximal segment rotates due to interaction of the links.
FIGS. 7A-7D are sequential views of the prosthetic digit of FIGS. 4A-4D shown in various rotated configurations where the middle and distal segments rotate independently of rotation of the proximal segment due to interaction of the links.
FIGS. 8A-8B are sequential views of an embodiment of an actuator that may be used in any of the prosthetic digits described herein, where the housing translates axially relative to the leadscrew.
FIG. 8C is a cross-sectional view of the actuator of FIG. 8A as taken along the line 8C-8C indicated in FIG. 8A.
FIGS. 9A-9B are sequential views of another embodiment of an actuator that may be used in any of the prosthetic digits described herein, where the housing translates axially relative to the leadscrew.
FIG. 9C is a cross-sectional view of the actuator of FIG. 9A as taken along the line 9C-9C indicated in FIG. 9A.
FIG. 9D is a cross-section view of the actuator of FIG. 9A and taken at a ninety degree angle with respect to the line 9C-9C indicated in FIG. 9A.
FIGS. 10A-10C are various views of an embodiment of a prosthetic digit, having articulating proximal, middle and distal segments and mechanically-connected rigid links, that may be used with the lower arm stump of FIGS. 1A-1B or prosthetic hand of FIGS. 2A-2B.
FIGS. 11A-11B are perspective and cross-sections views respectively of another embodiment of a prosthetic digit, having articulating proximal, middle and distal segments.
FIGS. 12A-12C are various views of the actuator of the prosthetic digit of FIGS. 11A-11B.
FIG. 13 is a perspective view of the actuator of FIGS. 12A-12C with some components removed for clarity.
FIG. 14 is a cross-sectional view of a portion of the prosthetic digit of FIGS. 11A-11B.

The foregoing and other features of the present disclosure will become more fully apparent from the following description and appended claims, taken in conjunction with the accompanying drawings. Understanding that these drawings depict only several embodiments in accordance with the disclosure and are not to be considered limiting of its scope, the disclosure will be described with additional specificity and detail through use of the accompanying drawings. In the following detailed description, reference is made to the accompanying drawings, which form a part hereof. In the drawings, similar symbols typically identify similar components, unless context dictates otherwise. The illustrative embodiments described in the detailed description, drawings, and claims are not meant to be limiting. Other embodiments may be utilized, and other changes may be made, without departing from the scope of the claims. It will be readily understood that the aspects of the present disclosure, as generally described herein, and illustrated in the drawing, can be arranged, substituted, combined, and designed in a wide variety of different configurations, all of which are explicitly contemplated and make part of this disclosure.

### DETAILED DESCRIPTION

The following detailed description is directed to certain specific embodiments of the development. In this description, reference is made to the drawings wherein like parts or steps may be designated with like numerals throughout for clarity. Reference in this specification to "one embodiment," "an embodiment," or "in some embodiments" means that a particular feature, structure, or characteristic described in connection with the embodiment is included in at least one embodiment of the invention. The appearances of the phrases "one embodiment," "an embodiment," or "in some embodiments" in various places in the specification are not necessarily all referring to the same embodiment, nor are separate or alternative embodiments necessarily mutually exclusive of other embodiments. Moreover, various features are described which may be exhibited by some embodiments and not by others. Similarly, various requirements are described which may be requirements for some embodiments but may not be requirements for other embodiments.

Features for prosthetic digits are described. The digits mimic natural fingers by having three articulating segments, including a proximal, middle and distal segment. The segments are articulated by an actuator and rotatably connected mechanical links configured to contribute to and/or cause rotation of the digit segments. Some versions may use one or more tendons to apply opening and closing forces to the digit. Other version may not need a tendon to effect articulation of the segments. Rotation of a proximal segment causes rotation of the middle and distal segments via mechanical interaction of the links. There may be a proximal link and a distal link. The digit may have an actuator that outputs linear actuation to cause rotation of the proximal segment and/or proximal link. The actuator may linearly translate a housing that is rotatably connected to the proximal segment at a joint. The housing pushes on the proximal segment at the joint to create a torque on the segment about an offset pivot. The pivot may be a pin attaching the proximal segment to the proximal link. The pivot is at a location offset from the joint. In some embodiments, the proximal link may be linearly expandable and retractable to allow for variable relative rotational positions of the digit segments. The distal digit segment may rotate independent of rotation of the proximal digit segment. The digit may thus have multiple degrees of freedom with only a single actuator. The rotated digit may provide articulation that mimics a natural finger and thus fully surrounds both small and large objects to provide and restore enhanced gripping functionality to amputees. The digit provides space, weight and power savings due to the need for only a single actuator. The segments may provide movement similar to movement of respective human phalanges in sound natural fingers. In some embodiments, the digit includes transmission features for a worm wheel rotation by a lead screw. A keyed member such as a central axle is spring-biased and transmits rotation from the worm wheel to the digit while allowing for manual rotation of the digit without damaging the worm wheel or other components.

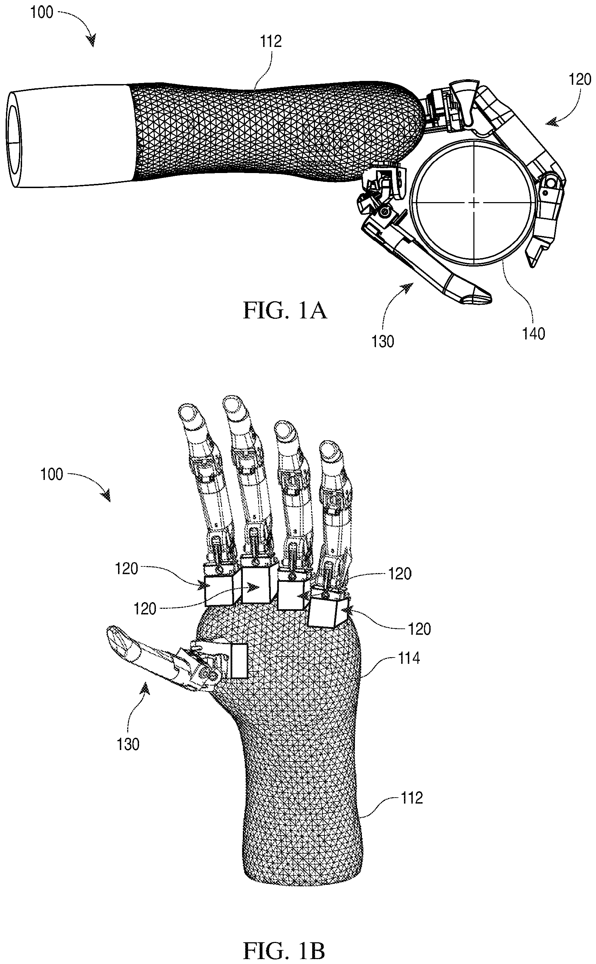

FIGS. 1A-1B are side and front views, respectively, of a lower arm prosthetic system 100 including a lower arm stump 112 having four prosthetic digits 120 and a prosthetic thumb 130 attached to the stump 112. FIG. 1A is a side view of the system 100. FIG. 1B is a front or palm-side view of the system 100. The prosthetic digits 120 and/or thumb 130 may be any of the prosthetic digits described herein. The digits 120 may be connected to the end of the lower arm stump 112, as shown in FIG. 1A, or to a residual natural palm 114, as shown in FIG. 1B.

As shown in FIG, 1A, the digits 120 and thumb 130 are grasping an object 140, shown as a round object such as a can or ball. The digits 120 are surrounding the object 140 such that the object 140 may be held securely by the system 100. The rotatable capability of the segments of the digits 120 allows for this secure grasp. The shape of the object 140 has a width and contour that allows the articulating digits 120 to provide a secure grasp. The digits 120 have various articulating segments that may rotate at various angles with respect to the adjacent segment. In some embodiments, the segments may rotate accordingly to a fixed angular relation, such that only certain sizes and shapes of objects 140 may be securely grasped. In some embodiments, the segments may rotate accordingly to a variable angular relation, such that only different sizes and shapes of objects 140 may be securely grasped.

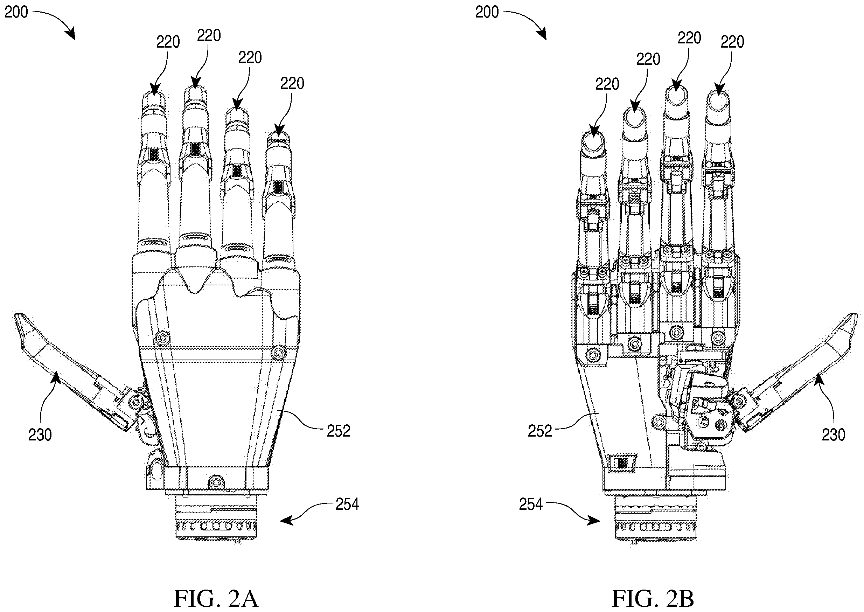

FIGS. 2A-2B are back and front views, respectively, of a prosthetic hand 200 incorporating embodiments of prosthetic digits 220 and a prosthetic thumb 230. The hand 200 has a palm portion 252 attached to proximal ends of the digits 220 and thumb 230. The hand 200 may have a wrist 254 that may rotate, which may allow for rotation of the palm portion 252, and the digits 220 and thumb 230 attached thereto, about a longitudinal axis defined by the wrist 254. The prosthetic digits 220 may be any of the prosthetic digits described herein. The prosthetic digits 220 may rotate according to a fixed or variable angular relation among the articulating digit segments, as described with respect to the system 100 of FIGS. 1A-1B.

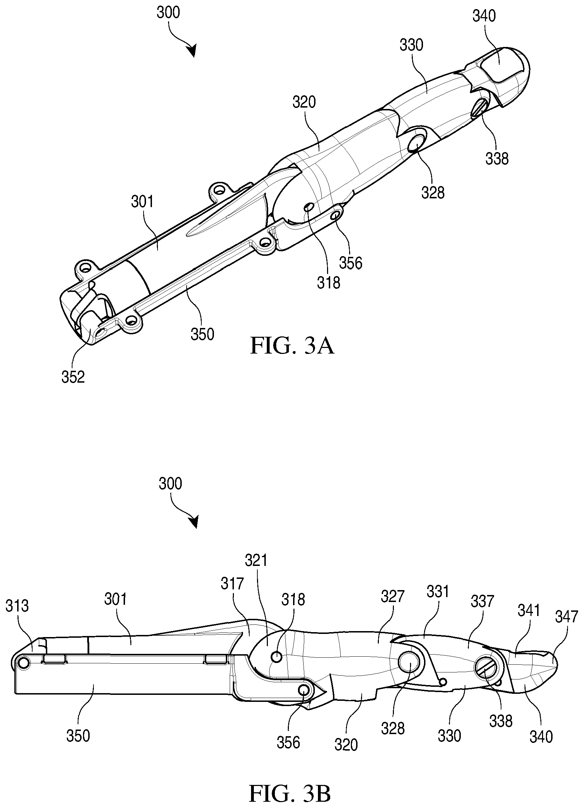

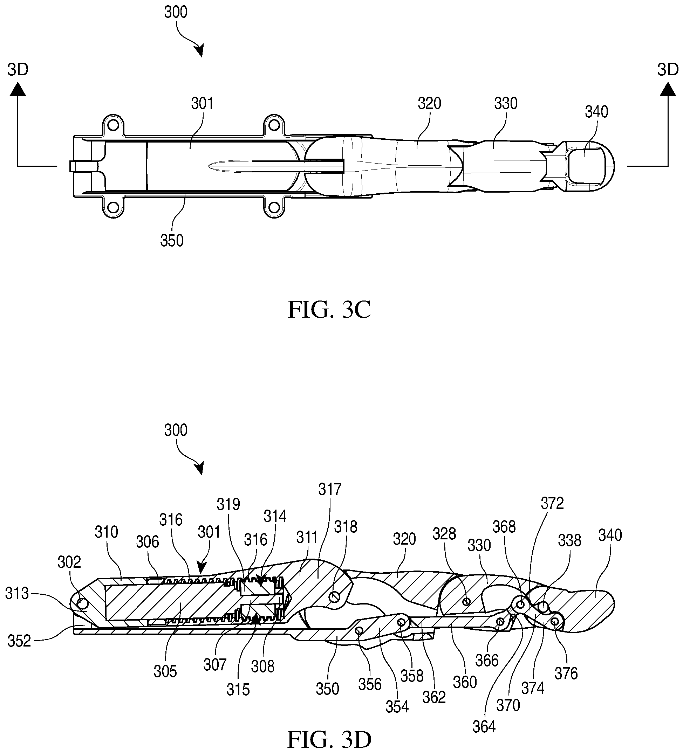

FIGS. 3A-3D are various views of an embodiment of a prosthetic digit 300. The digit 300 may be used with the system 100 or hand 200. The digit 300 includes an actuator 301, a mount 350, a proximal segment 320, a middle segment 330, and a distal segment 340. The segments may articulate, for example rotate, relative to each other. The digit 300 includes mechanically-connected links, which may be rigid, as further described herein, for example with respect to FIGS 3D-3G. The segments 320, 330, 340 may provide natural movement similar to that provided respectively by proximal, middle and distal phalanges of a sound natural finger.

The mount 350 and/or the actuator 301 may be connected with and/or located within, partially or completely, the arm stump 112, the residual palm 114, or the prosthetic palm 252. The proximal segment 320 may rotate relative to the mount 350 and/or the actuator 301. The middle segment 330 may rotate relative to the proximal segment 320. The distal segment 340 may rotate relative to the middle segment 330.

As shown in FIG. 3B, the actuator 301 includes a proximal end 313 and extends to a distal end 317. The proximal end 313 may attach to a hand, palm, etc. The distal end 317 attaches to a proximal end 321 of the proximal segment 320. The proximal segment 320 is rotatable relative to the actuator 301 about the joint 318. The actuator 301 may apply a normal force to the proximal segment 320 at the joint 318 to cause the proximal segment 320 to pivot about an offset first pivot 356, as further described herein. The proximal segment 320 extends from the proximal end 321 to a distal end 327. The distal end 327 attaches to a proximal end 331 of the middle segment 330. The middle segment 330 is rotatable relative to the proximal segment 320 about the joint 328. The middle segment 330 extends from the proximal end 331 to a distal end 337. The distal end 337 attaches to a proximal end 341 of the distal segment 340. The distal segment 340 is rotatable relative to the middle segment 330 about the joint 338. The rotatable connections at the joints 318, 328, 338 may include pin connections, hinges, and/or other suitable features for providing a rotatable engagement.

FIG. 3D is a cross-section view of the digit 300, as taken along the line 3D-3D indicated in FIG. 3C. As shown in FIG. 3D, the digit 300 may include the actuator 301. The actuator 301 may be a linear actuator. The actuator 301 produces or results in linear motion. As shown, the actuator 301 may include a motor 305 supplied with power from a battery, which may be in the hand or other location. A support 310, such as a motor mount or other structure, may carry or otherwise support the actuator 301. The support 310 may have an pin 302 or other suitable feature in a proximal end thereof to secure, for example rotatably attach, the support 310 with the mount 350.

The actuator 301 includes a housing 311. The housing 311 extends axially and defines a cavity 315 therein. The cavity 315 may be a cylindrical opening extending axially through the housing 311. A proximal end of the housing 311 may be open to the cavity 315. A distal end of the housing 310, for example at the distal end 37 of the actuator 301, connects with the proximal segment 320 at the joint 318. The housing 311 translates axially to cause rotation of the proximal segment 320, as further described herein.

The motor 305 may be supported, for example a fixed portion thereof, by the support 310. There may be a bushing 306 rotationally supporting a rotating portion of the motor 305, which may be located within and/or supported by the support 310. The motor 305 may include a shaft 307 extending therefrom, for example extending distally therefrom, that is rotated about an axis along which the shaft 307 extends. A cap 308, such as a nut, may attach to a distal end of the shaft 307. A leadscrew 314 having external threads 319 thereon may be positioned about the shaft 307 and secured in place by the cap 308. The leadscrew 314 may be a nut having external threads or other suitable features that engage corresponding internal structure of the housing 311 to translate the housing 311 back and forth.

The actuator 301 may output linear motion to cause rotation of the digit 300, as further described. The motor 305 or other portions of the actuator 301 may use or provide rotary, linear, cyclic and/or other types of motion. As shown, the actuator 301 is in mechanical communication with the leadscrew 314 having external threads 319. The actuator 301 rotates the leadscrew 314. The external threads 319 of the leadscrew 314 are in mechanical communication with internal threads 316 of the housing 311. The internal threads 316 may be located along the cavity 315 of the housing 311. The housing 311 may move relative to the support 310. The leadscrew 314 is rotated while remaining axially stationary to cause the housing 311 to translate axially along an axis defined by the cavity 315 via interaction of the external and internal threads 314, 316. The threaded engagement features and rotational motion of the actuator 301 is one example embodiment. Other features and/or actuator types may be used to output linear motion of the housing 311.

As the housing 311 is advanced distally and proximally, the actuator 301 may rotate about the pin 302 to accommodate the rotating proximal segment 320. For instance, the joint 318 may translate slightly during rotation, and the distal end of the housing 311 may move accordingly such that the actuator 301 rotates slightly at the pin 302. The actuator 301 may rotate counterclockwise as oriented in FIG. 3D during a distal movement of the housing 311 for a closing rotational movement of the segments 320, 330, 340. The actuator 301 may rotate clockwise as oriented in FIG. 3D during a proximal movement of the housing 311 for an opening rotational movement of the segments 320, 330, 340. Other configurations of the digit 300 may result in opposite rotations of the actuator 310 during opening and closing of the segments 320, 330, 340.

As further shown in FIG. 3D, the digit 300 includes a mount 350, a proximal link 360, and a distal link 370. The mount 350 extends from a proximal end 352 to a distal end 354. The proximal link 360 extends from a proximal end 362 to a distal end 364. The distal link 370 extends from a proximal end 372 to a distal end 374.

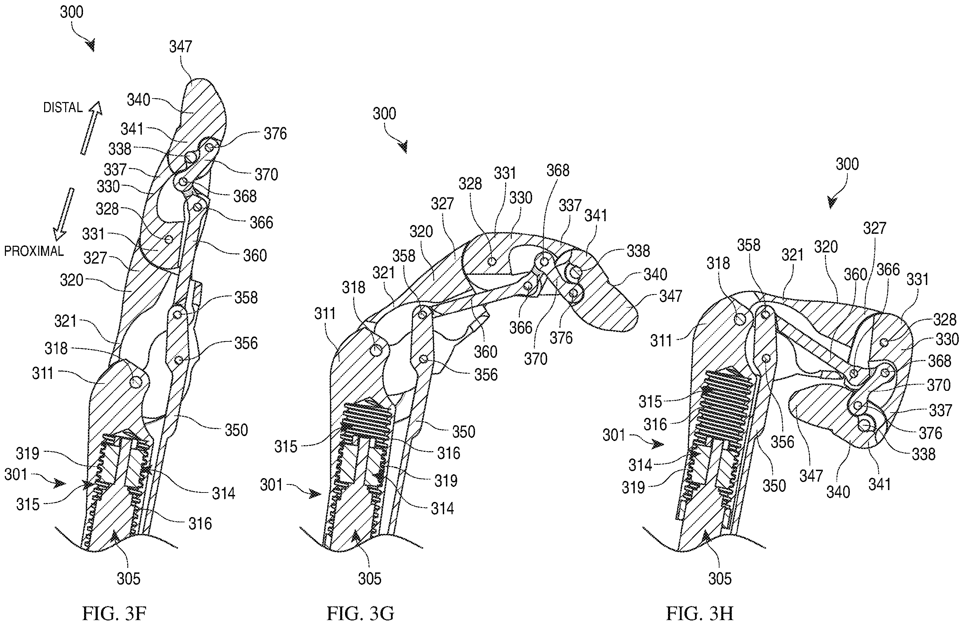

The proximal end 352 of the mount 350 may be attached to a proximal end of the actuator 301, for example rotatably attached thereto. The mount 350, such as at the proximal end 352 and/or other locations, may be attached to a hand, such as a prosthetic hand. Further details of the mount 350 are described herein, for example with respect to FIG. 3H. The distal end 354 of the mount 350 is rotatably attached to the proximal end 362 of the proximal link 360 about a connection 358. The mount 350 is also rotatably attached to the proximal segment 320 of the digit 300 about a first pivot 356. The first pivot 356 is located between the proximal and distal ends 352, 354 of the mount 350.

The proximal link 360 is rotatably attached to the middle segment 330 of the digit 300 about a second pivot 366. The second pivot 366 is located between the proximal and distal ends 362, 364 of the proximal link 360. The proximal link 360 may include a dogleg, where the proximal end 362 extends along a first axis and the distal end 364 extends a long a second axis that is at an angle relative to the first axis. The second pivot 366 may be located at or near the vertex of the dogleg of the proximal link 360. The distal end 364 of the proximal link 360 is rotatably attached to the proximal end 372 of the distal link 370 about a connection 368. The distal end 374 of the distal link 370 is rotatably attached to the distal segment 340 of the digit 300 about a third pivot 376.

In sum, the digit segments 320, 330, 340 are, respectively, rotatably attached to the links 320, 330, 340 at, respectively, the pivots 356, 366, 376. The segments 320, 330, 340 are rotatably attached to each other at the joint 318, which rotatably connects the proximal segment 320 to the middle segment 330, and at the joint 328, which rotatably connects the middle segment 330 to the distal segment 340. The links 350, 360, 370 are rotatably attached to each other at the connection 358, which rotatably connects the mount 350 to the proximal link 360, and at the connection 368, which rotatably connects the proximal link 360 to the distal link 370.

All or some of the rotatable connections at the joints 318, 328, 338, at the pivots 356, 366, 376, and at the connections 358, 368 may include pins, hinges, and/or other suitable features for providing a rotatable engagement. The axes of rotation for the joints 318, 328, 338, pivots 356, 366, 376, and connections 358, 368 may be perpendicular to a longitudinal axis of the digit 300. Such longitudinal axis may be defined by the fully extended digit 300, for example as shown in FIG. 3F. The longitudinal axis may be defined by the direction of linear movement provided by the actuator 301, for example the direction of linear movement of the leadscrew 314. The rotation axes for the joints 318, 328, 338, pivots 356, 366, 376, and connections 358, 368 may be parallel to each other. The locations of the joints 318, 328, 338, pivots 356, 366, 376, and connections 358, 368 may change as the digit 300 rotates, for example some or all of these the locations may change relative to the support 310 and/or relative to the mount 350.

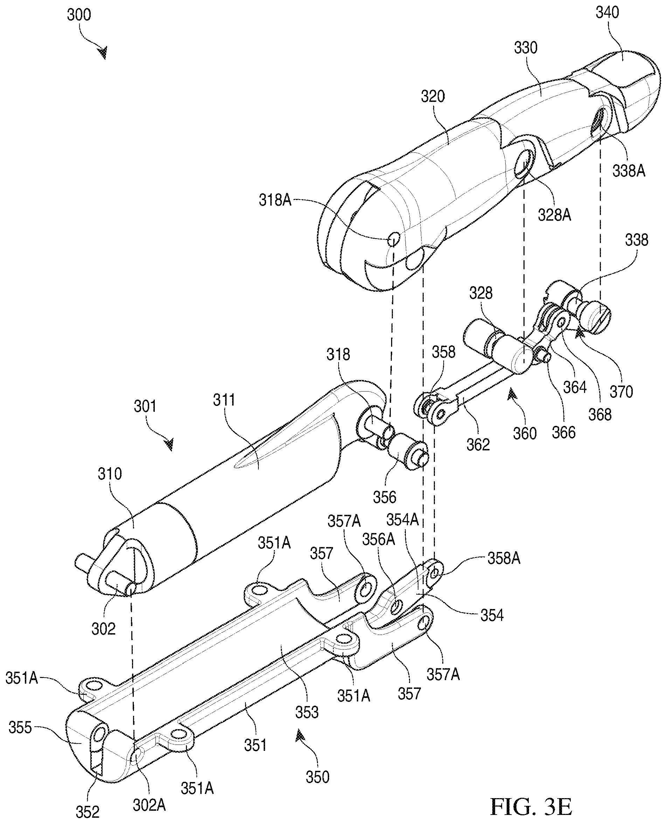

FIG. 3E is a partially exploded perspective view of the prosthetic digit 400. As shown, the mount 350 includes an elongated proximal portion 351 defining a cavity 353 therein. The proximal end 352 includes a proximal wall 355 having openings 302A extending therethrough. The pin 302 of the support 310 may extend through the openings 302A to rotatably connect the proximal ends of the actuator 301 and mount 351. This allows the actuator 301 to rotate slightly at the proximal end as needed for digit actuation. The mount 350 includes a series of tabs 351A to connect the mount 350 to a hand, such as the prosthetic hand 200 or the palm 114. The mount 350 may fixedly attach to the hand. There may be four tabs 351A as shown, or more or fewer than four. The mount 350 includes two distally extending forks 357. The forks 357 extend from the distal end of the portion 351. The forks 357 define a space therebetween that receives a proximal portion of the proximal segment 320. The forks 357 include openings 357A that receive therein the pivot 356. The pivot 356 is shown as a pin with rollers.

The mount 350 includes a prong 354A extending distally from the proximal end of the portion 351. The prong 354A is located between the forks 357. The prong 354A is at the proximal end 354 of the mount 350. The prong 354A includes an opening 356A therethrough that receives therein a central portion of the pivot 356. The pivot 356 may thus rotate within the openings 356A, 357A, and/or provide an axle about which the proximal segment 320 rotates. The prong 354A includes an opening 358A at a distal end thereof. The opening 358A receives therein the connection 358, shown as a pin. The connection 358 may thus rotate within the openings 358A, and/or provide an axle about which the proximal link 360 rotates, as described herein.

The actuator 301 includes the joint 318, shown as a pin. The joint 318 is received into openings 318A of the proximal segment 320. The joint 318 may be a shear pin that is pushed by the housing 311 axially to impart a force on the proximal segment 320 at the openings 318A. The joint 318 is offset from the pivot 356. Thus pushing on the joint 318 will create a torque on the proximal segment about the pivot 356. The axes of rotation of the joint 318 and pivot 356 may be parallel to each other.

The middle segment 330 includes one or more openings 328A which receives the joint 328 therein. The joint 328 is shown as a pin. The joint 328 may thus rotate within the openings 328A, and/or provide an axle about which the proximal and middle segments 320, 330 rotate, as described herein. The distal segment 340 includes one or more openings 338A which receives the joint 338 therein. The joint 338 is shown as a pin. The joint 338 may thus rotate within the openings 338A, and/or provide an axle about which the middle and distal segments 330, 340 rotate, as described herein.

FIGS. 3F-3H are sequential views of the prosthetic digit 300 shown in various rotated configurations. "Distal" and "proximal" as used herein have their usual and ordinary meaning. For clarity, the "distal" and "proximal" directions are indicated in FIG. 3F for the fully extended digit 300, and generally refer to a direction or portion of the digit 300 that is, respectively, farther from or closer to the proximal end 352 of the mount 350 along the length of the digit 300. FIG. 3F shows an embodiment of a fully straightened digit 300, FIG. 3G shows an embodiment of partially closed digit 300, and FIG. 3H shows an embodiment of a fully closed digit 300.

The middle and distal segments 330, 340 may rotate as the proximal segment 320 rotates due to interaction of the mount 350 and links 360, 370 as further described. As shown, for example in FIG. 3H, the distal segment 340 may completely close such that the distal segment 340 is parallel or near parallel with the proximal segment 320. In some embodiments, the distal segment 340 may rotate through this parallel position such that at full rotation the distal segment 340 is angled back toward the middle segment 320. The distal segment 340 may contact the proximal segment 320 in the fully rotated configuration. Such full or more complete closure of the distal segment 340 provides advantageous gripping capability with the digit 300 and more fully restores lost sound finger dexterity to a user, such as an amputee. The features described herein, such as the configuration and interaction of the mount 350, links 360, 370 and segments 320, 330, 340, among other things, contribute to such advantages.

To cause rotation of the digit 300, the actuator 301 may rotate the leadscrew 314 having the external thread. The external threads of the leadscrew 314 mechanically communicate with internal threads 316 of the housing 311. The actuator 301 may rotate the leadscrew 314 in a first rotational direction to cause the housing 311 to move, for example to translate, distally relative to the leadscrew 314. The leadscrew 314 may remain axially stationary. The housing 311 moves farther distally as shown sequentially from FIG. 3F to FIG. 3G to FIG. 3H. The direction of rotation of the digit 300 may be reversed (e.g., from FIG. 3H to FIG. 3G to FIG. 3F) by the actuator 301 rotating the leadscrew 314 in a second rotational direction, that is opposite to the first rotational direction, to cause the housing 311 to move, for example to translate, proximally relative to the leadscrew 314.

Distal movement of the housing 311 causes the proximal end 321 of the proximal segment 320 to move distally via the rotatable connection at the joint 318. Distal movement of the proximal segment 320 at the joint 318 will cause the proximal segment 320 to rotate clockwise (as oriented in the figures) about the first pivot 356 due to the offset locations of the joint 318 and the pivot 356. A line of action of force is imparted on the proximal segment 320 that extends through the joint 318 and thus imparts a moment on the proximal segment 320 about the pivot 356. The clockwise rotation of the proximal segment 320 about the first pivot 356 causes clockwise rotation of the proximal segment 320 relative to the housing 311 about the joint 318. Thus, the proximal segment 320 rotates clockwise as shown sequentially viewed from FIG. 3F to FIG. 3G to FIG. 3H. To reverse the direction of rotation in the counterclockwise direction (as oriented in the figures), these movements may be reversed, where the housing 311 is moved proximally to cause the proximal end 321 of the proximal segment 320 to move proximally and rotate counterclockwise about the first pivot 356 and the joint 318. A pinned or other type connection at the joint 318 as described herein may allow for such pushing and pulling forces by the housing 311 to be transferred to the proximal segment 320.

As the proximal segment 320 rotates clockwise about the pivot 356, the middle segment 330 also rotates clockwise with the rotating proximal segment 320 due to the connection of the two segments 320, 330 at the joint 328. In some embodiments, the middle segment 320 may be constrained from rotating farther in the counterclockwise direction, for instance the configuration shown in FIG. 3F may be the limit of rotation of the middle segment 330 relative to the proximal segment 320 about the j oint 328.

The rotation of the middle segment 330 also causes the distal segment 340 to rotate clockwise, due to the connection of the two segments 330, 340 at the joint 368. In some embodiments, the distal segment 340 may be constrained from rotating farther in the counterclockwise direction, for instance the configuration shown in FIG. 3F may be the limit of rotation of the distal segment 340 relative to the middle segment 330 about the joint 338.

As the middle segment 320 rotates clockwise, the proximal link 360 also rotates clockwise due to the connection of the middle segment 320 and the proximal link 360 at the second pivot 366. Further, the proximal link 360 is translationally constrained by the mount 350 at the rotatable connection 358. The proximal link 360 thus rotates clockwise about the connection 358. The joint 328 is offset from the second pivot 366 as shown. Thus a torque may be imposed on the middle segment 320 about the pivot 366. The axes of rotation of the joint 328 and second pivot 366 may be parallel.

As the proximal link 360 rotates clockwise about the connection 358, this also causes the distal link 370 to rotate clockwise due to the translational constraint between the proximal link 320 and the distal link 330 at the rotatable connection 368. As the distal link 330 rotates clockwise, the distal segment 340 is translationally constrained by the distal link 330 at the third pivot 376. The distal segment 340 also rotates relative to the middle segment 330 about the rotatable connection at the joint 338. The joint 338 is offset from the third pivot 376 as shown. Thus a torque may be imposed on the distal segment 340 about the pivot 376. The axes of rotation of the joint 338 and third pivot 376 may be parallel. The distal segment 340 thus rotates farther clockwise about the third pivot 376 to provide the closed configuration shown in FIG. 3H.

The digit 300 may be rotated in the counterclockwise direction sequentially from the configurations shown in FIG. 3H to FIG. 3G to FIG. 3F. The counterclockwise rotation operates in reverse as described above with respect to the clockwise rotation. For example, proximal movement of the proximal end 321 of the proximal segment 320 pulls proximally at the joint 318 and causes the proximal segment 320 to rotate counterclockwise about the pivot 356, which causes the middle segment 330 and proximal link 360 to rotate counterclockwise respectively about the joint 328 and pivot 366, which causes the distal segment 340 and distal link 370 to rotate counterclockwise respectively about the joint 338 and pivot 376.

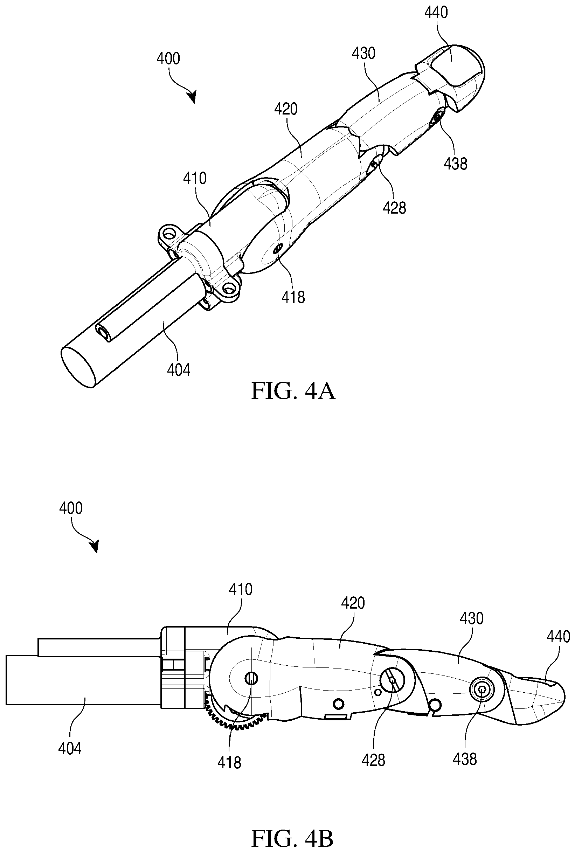

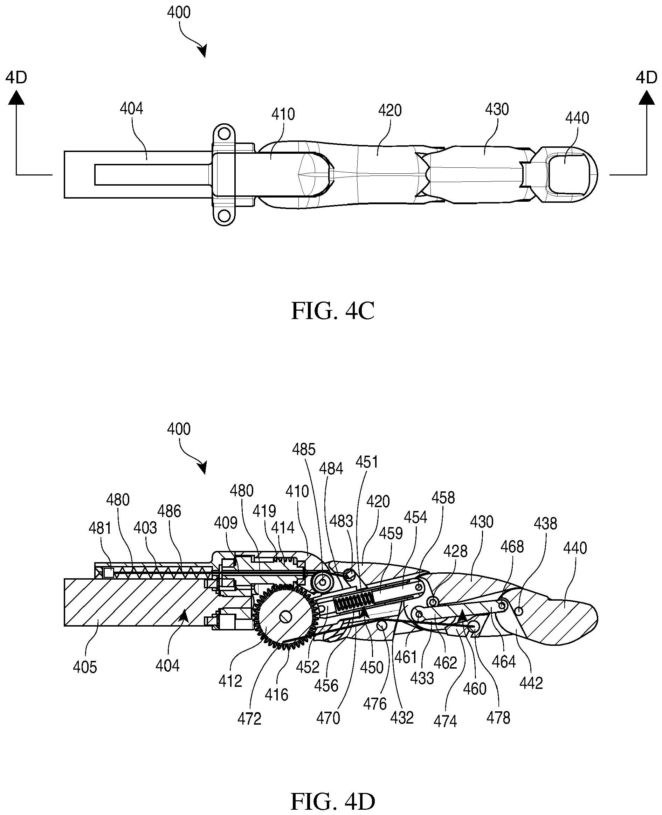

FIGS. 4A-4D are various views of another embodiment of a prosthetic digit 400. The digit 400 may be used with the system 100 or hand 200. The digit 400 includes a mount 410, a proximal segment 420, a middle segment 430, and a distal segment 440. The mount 410 and segments 420, 430, 440 may have the same or similar features and/or functions as the mount 350 and segments 320, 330, 340, and thus may articulate, for example rotate, relative to each other. The digit 400 includes mechanically-connected rigid links including an expandable proximal link 450, as further described herein, for example with respect to FIGS 4D-7D.

The mount 410 and segments 420, 430, 440 may be rotatably attached at joints 418, 428, 438, which may have the same or similar features and/or functions as the joints 318, 328, 338, respectively. However, the mount 410 may not have a linearly translatable portion. The digit 400 may have an actuator 404, which may have the same or similar features and/or functions as the actuator 301, except as otherwise described.

FIG. 4D is a cross-section view of the digit 400, as taken along the line 4D-4D indicated in FIG. 4C. As shown in FIG. 4D, the mount 410 may support the actuator 404. The actuator 404 may include a housing 403 extending proximally. The housing 403 may be used to house features for rotation of the segments 420, 430, 440, such as a spring 486 that provides a force in a proximal direction on a plunger 481 attached to a proximal end 482 of a return tendon 480, as further described herein. Some embodiments may not include the return tendon 480.

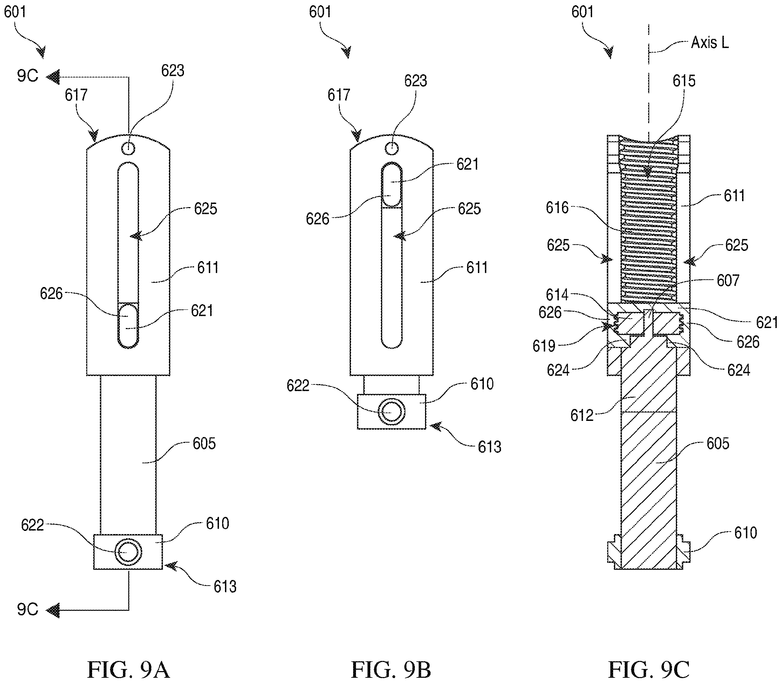

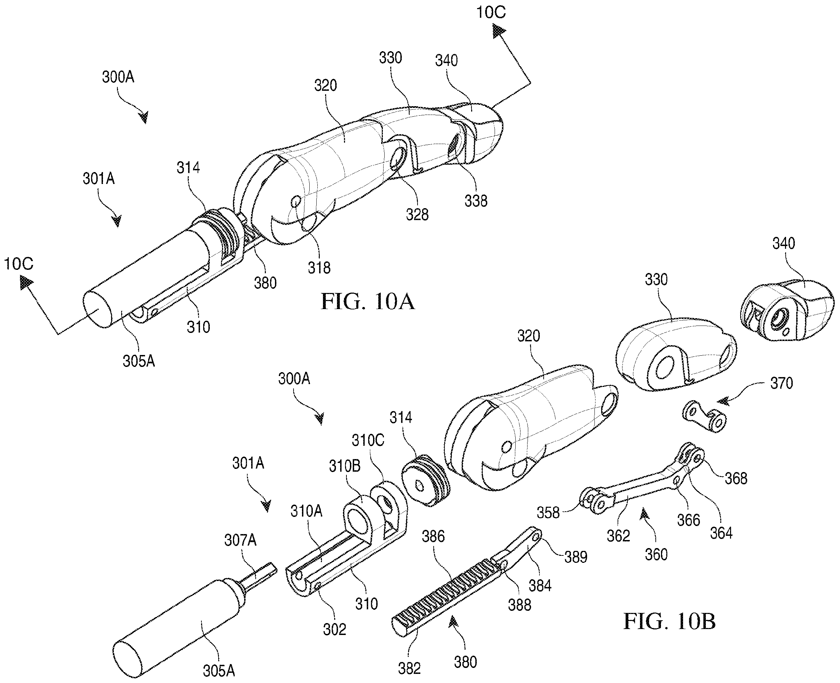

The actuator 404 may include a motor 405 supplied with power from a battery, which may be in the hand or other location. The motor 405 may be in mechanical communication with an output shaft 409 that extends, for example distally, therefrom. The motor 405 may rotate a first shaft at a distal end of the motor 405 and that is attached to a gear that mechanically communicates with a gear attached to the shaft 409. The gears may be mesh gears having teeth or cogs, or other suitable types of gears. Rotation of the motor shaft may rotate the shaft 409 via interaction of the corresponding gears. A worm gear 414 having external threads 419 thereon may be attached to the shaft 409. Actuation of the motor 405 causes motion to be transmitted via a gearbox to the shaft 409 to rotate the worm gear 414. Axial movement of the worm gear 414 may be restrained by a thrust bearing or by thrust bearing-like components on both axial sides of the worm gear 414. Other features as described herein may be used to axially constrain the worm gear 414, such as the projections 310B, 301C described with respect to FIGS. 10B-10C and/or the thrust bearing as described with respect to FIGS. 9A-9D. The digit 400 may include a worm wheel 412 having external teeth 416 thereon. The threads 419 of the worm gear 414 contact the teeth 416 of the worm wheel 412 to cause rotational motion of the worm wheel 412 about a first axis. The worm wheel 412 may be rotated a first rotational direction about the first axis to cause a first rotation of the digit 400 in a first direction (e.g. to close the digit 400). The worm wheel may have pulley features that attach to and wrapingly receive therearound a proximal end of an actuation tendon 470, as further described. The worm wheel 412 may be rotated in a second rotational direction about the first axis that is opposite the first rotational direction to allow for a second rotation of the digit 400 in a second direction that is opposite the first direction (e.g. to open the digit), which movement may be caused by the return tendon 480, as further described. Some embodiments may not include the actuation tendon 470 or return tendon 480.

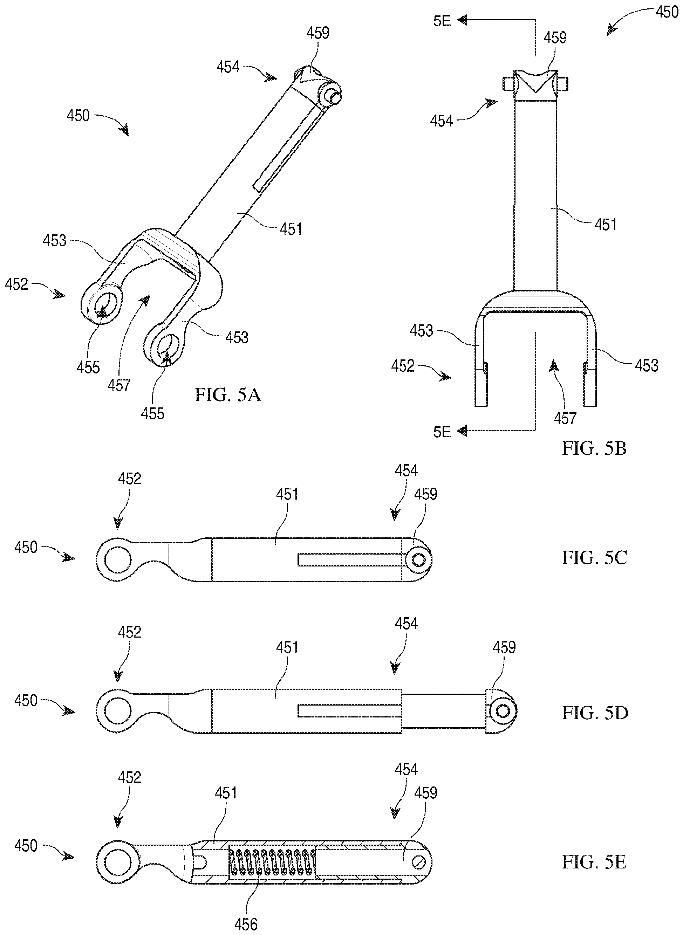

The digit 400 includes an expandable proximal link 450. The link 450 is attached to the worm wheel 412. Rotation of the worm wheel 412 in a first rotational direction for a first angular amount causes a corresponding rotation of the link 450 in the first rotational direction for the first angular amount. The link 450 may expand. The link 450 or a portion thereof may extend distally relative to the worm wheel 412. The link 450 includes a proximal end 452 and extends to a distal end 454. The proximal end 452 includes a fixed portion 451, such as a cylinder. The distal end 454 includes a housing 459, such as a piston. The link 450 may include a spring 456, such as an extension spring. Extension of the spring 456 beyond a neutral length may cause a restoring force that biases the spring back to a shorter length. The link 450 may expand as it is rotated to allow for multiple degrees of freedom rotation of the digit 40. The housing 459 may expand distally relative to the fixed portion 451. The spring 456 may bias the housing 459 in the proximal direction. The housing 459 may retract in the proximal direction relative to the fixed portion 451. Further details of the link 450 are described herein, for example with respect to FIGS. 5A-5E.

The link 450 is attached to the middle segment 430 of the digit 400. The distal end 454 of the link 450 may be rotatably attached to the middle segment 430 at the connection 458. The middle segment 430 may include an ear 432 that rotatably connects with the link 450. The connection 458 may include a pin or other feature that extends through the link 450 and ear 432 at the connection 458. The link 450 may extend between two of the ears 432, with one ear 432 on either lateral side of the distal end 454 of the link 450 at the connection 458.

The digit 400 may include a distal link 460. The distal link 460 extends from a proximal end 462 to a distal end 464. The proximal end 462 may be rotatably attached to the ear 432 at a connection 461. The ear 432 may include a rounded slot 433. The connection 461 may include a pin or other feature that extends through the link 460 and rounded slot 433 at the connection 461. The connection 461 allows the proximal end 462 of the distal link 460 to rotate within and move along the slot 433 as the digit 400 articulates, for example as the middle segment 430 rotates relative to the proximal segment 420 and/or as the distal segment 440 rotates relative to the middle segment 430.

The distal link 460 is attached to the distal segment 440. The distal end 464 of the distal link 460 may be rotatably attached to the distal segment 440 at the connection 468. The connection 468 may include a pin or other feature that extends through the distal link 460 and distal segment 440 at the connection 468. The distal segment 440 may include an ear 442 having an opening therethrough and with which the distal link 460 is attached. The distal end 464 of the link 460 may extend between two of the ears 442, with one ear 442 on either lateral side of the distal end 464 of the link 460 at the connection 468.

FIGS. 5A-5E are various views of the proximal expandable link 450. FIG. 5A is a perspective view of the link 450, FIG. 5B is a top view, FIG. 5C is a side view in an unexpanded configuration, FIG. 5D is a side view in an expanded configuration, and FIG. 5E is a cross-section view as taken along the line 5E-5E shown in FIG. 5B.

The proximal link 450 may include an extension 453. There may be two extensions 453 extending proximally, for example forming a clevis type connection. The extensions 453 may each include an opening 455 therethrough. The extensions 453 may define a space 457 therebetween. The extensions 453 may laterally surround the worm wheel 412 when installed with the worm wheel 412 located in the space 457, and a pin or other feature may extend through the openings 455 and a central opening of the worm wheel 412 to connect the link 450 with the worm wheel 412.

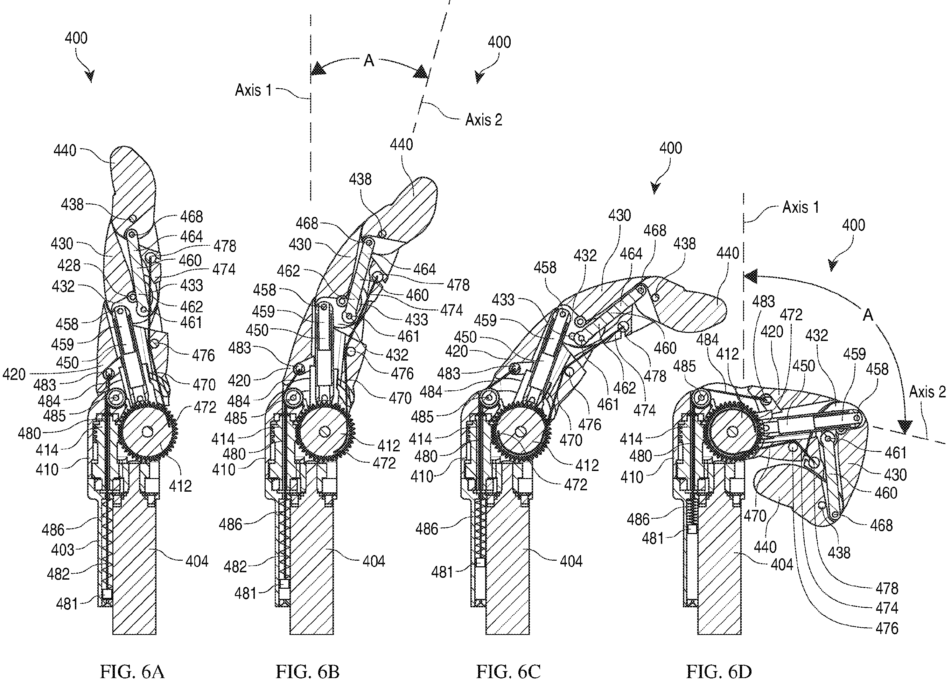

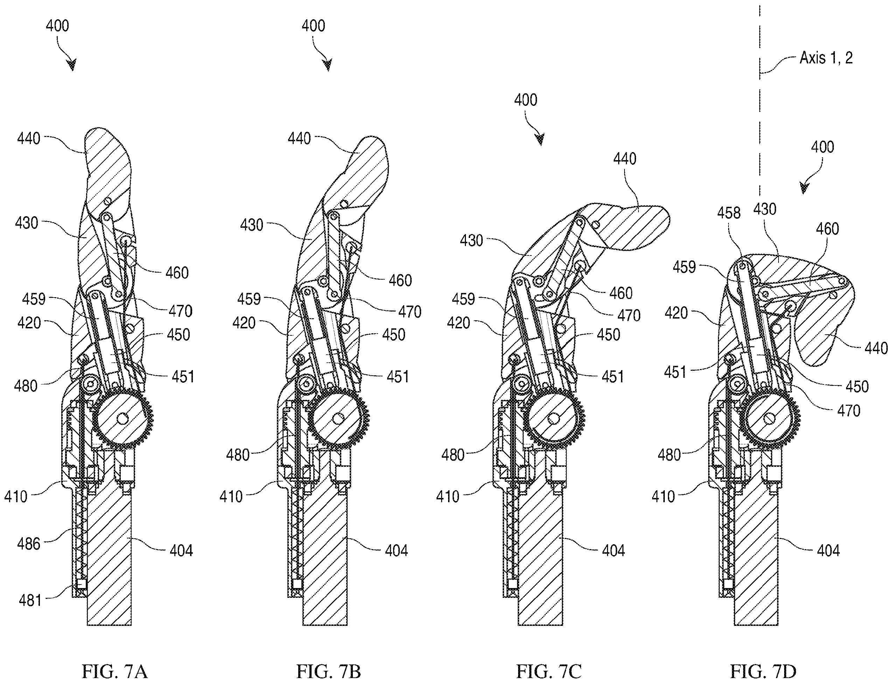

The housing 459 may move linearly with respect to the fixed portion 451. The fixed portion 451 may define a longitudinal axis along which the housing 459 may translate. A spring 456 may be located within the link 450. As shown in FIG. 5E, a proximal end of the spring 456 may be located within the fixed portion 451 and be attached to a proximal end of the link 450. A distal end of the spring 456 may attach to a proximal end of the housing 459. In some embodiments, the spring 456 may extend through and attach to the housing 459. FIG. 5D shows the link 450 expanded relative to the configuration in FIG. 5C. The expanded housing 459 will stretch the spring 456. The spring 456 will exert a restoring force on the housing 459 and bias the housing proximally. The link 450 may then return to the configuration shown in FIG. 5C. The link 450 may repeatedly extend and retract as the finger is rotated to close the digit 400 and then rotated back to open the digit 400. The link 450 may therefore expand during rotation of the digit 400, as further described herein, for example with respect to FIGS. 6A-6D. In some embodiments, the link 450 may not expand during rotation of the digit 400 for added degrees of freedom, as further described herein, for example with respect to FIGS. 7A-7D.

FIGS. 6A-6D are sequential views of the prosthetic digit 400 shown in various rotated configurations. The sequential views illustrate an embodiment of the middle and distal segments 430, 440 rotating as the proximal segment 420 also rotates. The rotation of the segments 420, 430, 440 may be due to the configuration and interaction of the mount 410, segments 420, 430, 440 and links 450, 460.

The proximal segment 420 may rotate relative to the mount 410 about the joint 418 (see FIGS. 4A-4B). To initiate rotation of the digit 400, the actuator 404 may cause the worm gear 414 to rotate and thereby rotate the worm wheel 412 about the first axis.

In some embodiments, the link 450 may rotate about the first axis with the rotating worm wheel 412. The link 450 may rotate the same or similar angular amount as the angular amount that the worm wheel 412 rotates. For example, rotation of the worm wheel 412 by fifteen degrees clockwise may cause a corresponding fifteen degree rotation of the link 450, etc.

In some embodiments, rotation of the link 450 may cause the proximal segment 420 to rotate. For example, the link 450 may be attached with the proximal segment 420, such that rotation of the link 450 in a first or second rotational direction may cause a corresponding rotation of the proximal segment 420 in the first or second rotational direction, respectively.

In some embodiments, rotation of the worm wheel 412 may not cause the link 450 or proximal segment 420 to rotate. For example, the link 450 may be rotatably connected to the worm wheel. The middle and distal segments 430, 440 may thus rotate while the proximal segment 420 does not rotate or rotates less as compared to a full rotation, as further described with respect to FIGS. 7A-7D. In some embodiments, actuation of the digit segments may be provided by the actuation tendon 470 attached to the worm wheel 412 and to the various segments 420, 430, 440, such that rotation of the worm wheel 412 will cause the tendon to pull in (shorten) to cause rotation of the segments 420, 430, 440. The return tendon 480 may rotate the digit 400 in the opposite direction, as described herein, and the worm wheel 412 may rotate in the opposite direction to allow the actuation tendon to pay out (lengthen). Thus, in some embodiments, the worm wheel 412 may rotate about the first axis while a proximal end of the link 450 does not rotate about the first axis.

The digit 400 may include the actuation tendon 470. The tendon 470 extends from a proximal end 472 attached to the worm wheel 412 to a distal end 474 attached to an attachment 478 of the middle segment 430. The tendon 470 extends distally from the worm wheel 412 and around an idler 476, such as a pulley, which may or may not rotate, and that is connected to the proximal segment 420. As the worm wheel 412 rotates clockwise as oriented from FIG. 6A to FIG. 6D (also shown in FIGS. 7A to 7D), the proximal end 472 of the tendon 470 wraps around the worm wheel 412. The tendon 470 effectively shortens in length and thus pulls on the attachment 478 and applies a force on the idler 476, causing the middle and proximal segments, to which the attachment 478 and idler 476 are respectively attached, to rotate in the clockwise direction as oriented.

The digit 400 may include the return tendon 480. The return tendon 480 extends from a proximal end 481 attached to the plunger 481. The plunger 481 is biased in the proximal direction by a compression spring 486 inside the housing 403. The tendon 480 extends from the housing 403 in a distal direction around an idler 485, such as a pulley, which may or may not rotate, to a distal end 484 of the tendon 480 attached to an attachment 483 of the proximal segment 420. As the proximal segment 420 rotates clockwise as oriented, due to the actuation tendon 470 as described, the attachment 483 pulls on the return tendon 480 causing the plunger 481 to move distally and compress or further compress the spring 486. The spring 486 compresses further as the digit 400 rotates further clockwise. The spring 486 thus applies a biasing force in the proximal direction to the plunger 481, biasing the tendon 480 in the proximal direction, and applying an opening or counterclockwise force to the proximal segment 420 via the attachment 483. In some embodiments, the spring 486 may be a constant force spring to apply a constant return force to the segment 420 in various rotational positions.

As the worm wheel 412 is rotated counterclockwise as oriented to effectively lengthen or pay out the actuation tendon 470, the biasing force on the return tendon 480 causes the proximal segment 420 to rotate open, or in the counterclockwise direction as oriented. Further, the spring-loaded expandable link 450, as described herein, then pulls proximally on the middle segment 430 at the connection 458 to rotate the middle segment 430 counterclockwise about the joint 428. The ear 432 may then rotate counterclockwise about the joint 428 to rotate the connection 461 of the distal link 460 counterclockwise about the joint 428 to rotate the distal segment 440 counterclockwise as well.

The tendons 470, 480 are just one example of how to effect articulation of the segments 420, 430, 440 in the prosthetic digit 400 having the expandable link 450. Some embodiments of the digit 400 having the expandable link 450 may not include the actuation tendon 470 and/or the return tendon 480. For example, features other than tendons may be used, such as other links, connections, joints, segments, etc. Therefore, the embodiments shown and described herein for articulation of the segments 420, 430, 440 are merely example embodiments of how the prosthetic digit 400 with the expandable link 450 may be implemented.

As the link 450 rotates, the rotatable connection 458 of the link 450 with the middle segment 430 translates or sweeps a rotational path. The middle segment 430 is translationally constrained with the distal end 459 of the link 450 at the connection 458. The middle segment 430 thus rotates relative to the link 450 about the connection 458 as the middle segment 430 is rotating to open or close the digit 400. The middle segment 430 also rotates relative to the proximal segment 420 about the joint 428 (see FIGS. 4A-4B).

As the middle segment 430 rotates, the connection 461 at the proximal end 462 of the distal link 460 moves along the slot 433. The connection 461 may include a pin sliding along the slot 433. This allows the ear 432 to rotate relative to the distal link 460. The distal link 460 thus rotates relative to the middle segment 430. As the distal link 460 rotates, the distal segment 440 also rotates due to the connection 468 between the distal link 460 and the distal segment 440. The distal segment 440 rotates relative to the middle segment 430 about the joint 438.

As shown in FIGS. 6B and 6D, the mount 410 or a portion thereof may extend along an Axis 1. The proximal segment 420 may extend along an Axis 2. The Axes 1,2 may form an angle A between them. The angle A may be the angular configuration of the proximal segment 420 relative to the mount 410. The angle A may range from zero degrees (e.g., in FIG. 6A) to ninety degrees or more (e.g., in FIG. 6D). In some embodiments, the angle A may be negative fifteen, negative ten, negative five, zero, five, ten, fifteen, twenty, twenty-five, thirty, thirty-five, forty, forty-five, fifty, fifty-five, sixty, sixty-five, seventy, seventy-five, eighty, eighty-five, ninety, ninety-five, one hundred, one hundred five, one hundred ten, or one hundred fifteen degrees, or other lesser, greater or in between angular amounts. The various values for the angle A may apply to any of the articulated configurations of the prosthetic digit 400 shown in any of FIGS. 6A-6D and other configurations.

The angle A may change as the digit 400 rotates, for example as the middle and distal segments 430, 440 rotate. As shown, the angle A may increase from the relatively open configuration of FIG. 6B to the relatively closed configuration of FIG. 6D, and vice versa. The angle A may be dependent on the amount of rotation of the middle and distal segments 430, 440, or vice versa. In some embodiments, the angle A may not change as the digit 400 rotates, for example as the middle and distal segments 430, 440 rotate. For example, the angle A may not change from the relatively open configuration of FIG. 6B to the relatively closed configuration of FIG. 6D, and vice versa. In some embodiments, the angle A may change by a small amount from the relatively open configuration of FIG. 6B to the relatively closed configuration of FIG. 6D, and vice versa, for example by five degrees or less, ten degrees or less, fifteen degrees or less, or twenty degrees or less. The angle A therefore may not be dependent on the amount of rotation of the middle and distal segments 430, 440, or vice versa, as further described herein, for example with respect to FIGS. 7A-7D.

The digit 400 may rotate as described to have the closed configuration shown in FIG. 6D. The Axis 2 along which the proximal segment 420 extends may be at about ninety degrees to the Axis 1. The middle segment 420 may be rotated to about parallel with the Axis 1. In some embodiments, the middle segment 420 may not be parallel with the Axis 1 in the closed configuration. As also shown, the distal segment 440 is rotated clockwise to be adjacent to the proximal segment 420. The segments 420, 430, 440 may thus rotate to provide a small closed grip with the digit 400.

FIGS. 7A-7D are sequential views of the prosthetic digit 400 performing a rotation with added degrees of freedom. The digit 400 is shown in various rotated configurations where the middle and distal segments 430, 440 rotate independently of rotation of the proximal segment 420 due to interaction of the links 450, 460. The digit 400 may rotate similarly as described with respect to FIGS. 6A-6D, except as otherwise described.

In some embodiments, the digit 400 may rotate to grab or cover an object having an irregular outer surface or contour. The rotational path of the digit 400 shown in FIGS. 6A-6D may not adequately cover or grasp the object due to the irregular outer surface. Thus the proximal and/or middle segments 420, 430 may be prevented from rotating clockwise beyond an angular amount. In such case, the middle and/or distal segments 430, 440 may continue to rotate to provide the desired functionality. FIGS. 7A-7D shown an example embodiment of rotation of the digit 400 where the proximal segment 420 does not rotate or does not completely rotate clockwise, while the middle and distal segments 430, 440 rotate clockwise.

As the digit 400 rotates from FIG. 7A to FIG. 7D, the proximal segment 420 may be prevented from rotation. This may be due to a force exerted on the proximal segment 420 by an outside object that counteracts the closing direction, such as contact with a part of the object the digit 400 is grasping. The middle and distal segments 430, 440 may continue to rotate due to the link 450 expanding. The link 450 as shown may elongate as the digit 400 rotates. The housing 459 may extend distally away from or proximally toward the fixed portion 451 as the digit 400 is rotated clockwise or counterclockwise, respectively. As shown in FIG. 7D, the angle A between the Axes 1 and 2 may therefore not change, or may change by a small amount, as described herein, for example with respect to FIGS. 6A-6D.

The link 450 may have a first axial length in FIG. 7A for instance where the digit 400 is straightened out, a second axial length in FIG. 7B where the digit 400 has partially rotated, a third axial length in FIG. 7C where the digit 400 is rotated farther but not completely, and a fourth axial length in FIG. 7D where the digit 400 is fully rotated. The first length may be shorter than each of the second, third and fourth lengths. The second length may be shorter than each of the third and fourth lengths. The third length may be shorter than the fourth length.

The middle and distal segments 430, 440 rotate as described with respect to FIGS. 6A-6D. The expanding and retracting link 450 allows the middle and distal segments 430, 440 to rotate without rotation or full rotation of the proximal segment 420. In some embodiments, the link 450 may not rotate. In some embodiments, the link 450 may partially rotate. In some embodiments, a tendon may be used to cause rotation of the middle and distal segments 430, 440 when the proximal segment 420 does not rotate or does not fully rotate. A tendon may be attached to the worm wheel 412 to cause rotation, as described with respect to FIGS. 6A-6D.

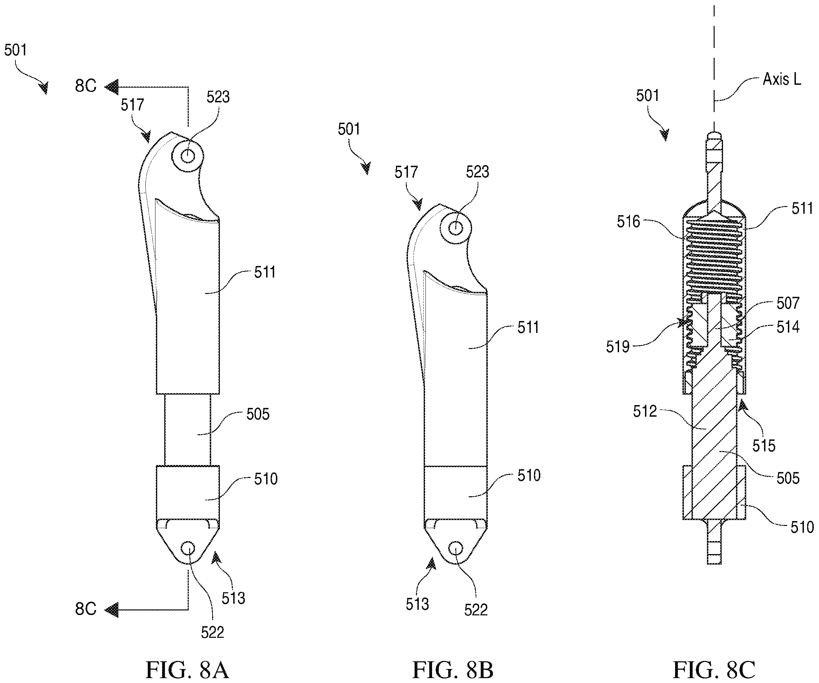

FIGS. 8A-8C are various views of an embodiment of an actuator 501 that may be used with the various prosthetic digits described herein. FIG. 8A is a side view of the actuator 501 in an extended configuration. FIG. 8B is a side view of the actuator 501 in a retracted configuration. FIG. 8C is a cross-section view of the actuator 501, as taken along the line 8C-8C indicated in FIG. 8A. The actuator 501 may be used in any of the prosthetic digits disclosed herein, such as the prosthetic digits of FIGS. 1A-3H. The actuator 501 may have the same or similar features and/or functions as the actuators 301, 404, and vice versa, except as otherwise described.

As shown in FIG. 8A, the actuator 501 includes a proximal end 513 and extends to a distal end 517. The proximal end 513 may attach to a hand, palm, etc. The distal end 517 may attach to a proximal end of a proximal segment of a prosthetic digit.

Advantageously, the actuator 501 is compact. The actuator 501 is small enough to fit at least partially within a prosthetic digit. The actuator 501 may be sized to fit in a proximal end of a prosthetic digit for a typical sized hand prosthetic. A prosthetic hand may include multiple actuators 501, for example one of the actuators 501 in each of its prosthetic digits. In some embodiments, in a closed or retracted configuration, the actuator 501 has an overall volume of no more than 11,550 mm³ (millimeters cubed). In some embodiments, in a closed or retracted configuration, the actuator 501 has a maximum length of no more than 75 mm (millimeters) and a maximum width of no more than 14 mm. In a closed or retracted configuration, the actuator 501 may have an overall volume of no more than 11,550 mm³, no more than 5,000 mm³, no more than 7,500 mm³, no more than 10,000 mm³, no more than 12,500 mm³, or no more than 15,000 mm³. In a closed or retracted configuration the actuator 501 may have a maximum length of no more than 75 mm (millimeters), no more than 25 mm, no more than 50 mm, no more than 100 mm, or no more than 125 mm, and/or a maximum width of no more than 14 mm, no more than 8 mm, no more than 10 mm, no more than 12 mm, no more than 16 mm, no more than 18 mm, or no more than 20 mm. The actuator 501 may have an aspect ratio, defined as the ratio of a maximum length in a retracted state to a maximum width in a retracted state, of no less than 1.5, no less than 2, no less than 2.5, no less than 3, no less than 3.5, no less than 4, no less than 4.5, or no less than 5.

In an open or extended configuration, in some embodiments the actuator 501 may have an overall volume of no more than 14,164 mm³. The actuator 501 may have a maximum length of 92 mm and/or a maximum width of 14 mm. In an open or extended configuration, the actuator 501 may have an overall volume of no more than 10,000 mm³, no more than 12,000 mm³, no more than 13,000 mm³, no more than 13,500 mm³, no more than 14,000 mm³, no more than 14,500 mm³, no more than 15,000 mm³, or no more than 16,000 mm³. In an open or extended configuration, the actuator 501 may have a maximum length of no more than 50 mm, no more than 60 mm, no more than 70 mm, no more than 80 mm, no more than 90 mm, no more than 95 mm, no more than 100 mm, or no more than 110 mm. In an open or extended configuration, the actuator 501 may have a maximum width of no more than 5 mm, no more than 8 mm, no more than 10 mm, no more than 12 mm, no more than 14 mm, no more than 16 mm, no more than 18 mm, no more than 20 mm, or no more than 25 mm.

The actuator 501 is a linear actuator that extends and retracts linearly. The actuator 501 produces or results in linear motion. As shown, the actuator 501 includes a motor 505 and a gearbox 512. The motor 505 is in mechanical communication with the gearbox 512. The motor 505 is supplied with power from a battery, which may be in the hand or other location. The actuator 501 includes a shaft 507 extending axially and distally from the gearbox 512 and/or motor 505. The actuator 501 may include a leadscrew 514 coupled to the motor 505 (e.g., coupled to the shaft 507). The leadscrew 514 may have an external thread 519 that is compatible with the threads of other components in the actuator 501. The leadscrew 514 may be or include a worm gear.

The actuator 501 includes a support 510. The support 510, such as a motor mount or other structure, may carry or otherwise support the actuator 501 and/or the motor 505. The support 510 may be configured to attach the actuator 501 to a hand. For example, the support 510 may be configured to receive a pin or other suitable feature in the proximal end thereof to secure, for example rotatably attach, the support 510 with a mount (e.g., a mount on a hand, palm, etc.). The support 510 may include a connector portion 522, such as an opening as illustrated in FIG. 8B, in the proximal end 513 which may receive the pin or other suitable feature. The actuator 501 may rotate about an axis defined by the connector portion 522 as the actuator 501 extends and retracts linearly, as described herein.

The actuator 501 includes a housing 511. The housing 511 extends and retracts axially. The housing 511 defines a cavity 515 therein. The cavity 515 may be a cylindrical opening extending axially inside or through the housing 511. The cavity 515 may have a maximum length and a maximum diameter. The ratio of the maximum length to the maximum diameter may be no less than 1.5, no less than 2, no less than 2.5, no less than 3, no less than 3.5, no less than 4, no less than 4.5, or no less than 5. The ratio of maximum length to maximum diameter may be from 3-6. The cavity 515 may be configured to receive therein the gearbox 512, shaft 507, leadscrew 514, and/or at least a portion of the motor 505. The housing 511 may have an internal thread 516. For example, as shown, the internal thread 516 may be located along the cavity 515 of the housing 511. A proximal end of the housing 511 may be open to the cavity 515. A distal end of the housing 511 may correspond to the distal end 517 of the actuator 501. The distal end of the housing 511 may connect with a proximal segment of a prosthetic digit at a j oint. For example, as shown in FIG. 8A, the distal end of the housing 511 may include an opening 523 that is configured to engage a proximal portion of a prosthetic digit. The actuator 501 may rotate about an axis defined by the opening 523 as the actuator 501 extends and retracts linearly, as described herein.

The actuator 501 may include an axially fixed portion and an axially movable portion. The fixed and moveable portions may be respectively fixed and moveable with respect to a longitudinal axis of the actuator 501, such as the axis L shown in FIG. 8C. The axially movable portion of the actuator 501 may slidably engage an outer surface of the fixed portion. The axially movable portion may include the housing 511. The axially fixed portion may include the motor 505, the gearbox 512, the support 510, the leadscrew 514, and the shaft 507.

The actuator 501 may output linear motion to cause rotation of a prosthetic digit. For example, the housing 511 may translate axially to cause rotation of the proximal segment of the prosthetic digit. The motor 505 or other portions of the actuator 501 may use or provide rotary, linear, cyclic and/or other types of motion.

The motor 505 may rotate the leadscrew 514 about the longitudinal Axis L (shown in FIG. 8C). The shaft 507 may be configured to be rotated by the motor 505 and be configured to rotate the leadscrew 514 about the longitudinal Axis L in a first rotational direction and a second rotational direction opposite the first rotational direction. The internal thread 516 of the housing 511 may be configured to at least partially engage with the external thread 519 of the leadscrew 514 such that rotation of the leadscrew 514 causes the housing 511 to translate axially along the longitudinal Axis L relative to the leadscrew 514 and/or the support 510 while the leadscrew 514 remains axially stationary. For example, the external thread 519 of the leadscrew 514 may be in mechanical communication with the internal thread 516 of the housing 511. Rotation of the leadscrew 514 in the first rotational direction causes the housing 511 to translate distally relative to the leadscrew 514 and/or the support 510 and rotation of the leadscrew 514 in the second rotational direction causes the housing 511 to translate proximally relative to the leadscrew 514 and/or the support 510.

The actuator 501 may be used in a prosthetic digit having a base configured to attach to a hand, a proximal segment, a middle segment, and a distal segment, an expandable link, and a wheel, such as a worm wheel or other rotatable member. The wheel may be placed in mechanical communication with the actuator 501. The actuator 501 may be configured to cause the wheel to rotate. The prosthetic digit may include a tendon extending distally from the wheel, a pulley rotationally connected to the proximal segment, and a tendon attachment coupled to the middle segment, as described herein, for example with respect to FIGS. 6A-7D. When the actuator 501 is used in a prosthetic digit, translation of the housing 511 in a distal direction along the longitudinal Axis L of the actuator 501 relative to the leadscrew 514 may cause the proximal segment of the prosthetic digit to rotate about a joint, which may cause the middle and distal segments to rotate. Rotation of the wheel by the actuator 501 in a first rotational direction may pull the tendon proximally and cause the distal segment of the prosthetic digit to rotate relative to the middle segment, as described.

The actuator 501 may be used in a variety of other prosthetic digits. The examples provided herein are only some embodiments. The compactness of the actuator 501 allows it to be used in prosthetic hands for each of the prosthetic digits, for example one, two, three, four or five actuators 501 may be used for each of a corresponding prosthetic digit. The actuator 501 may be housed entirely or partially within the prosthetic digit. The actuator 501 may be housed entirely or partially within the hand.

Further, in some embodiments, the actuator 501 may be assembled in a flipped orientation as that described herein. For example, the actuator 501 may be flipped in a proximal to distal direction such that the moveable and fixed portions are reversed. The housing 511 may be axially stationary while the motor 505 and other parts may move axially. The housing 511 may be attached to the prosthetic hand and the motor 505 for instance the motor mount 510 may be attached to the prosthetic digit, for instance the proximal segment. Thus the general principles of the actuator 501 described herein may be used in a variety of contexts that are within the scope of the disclosure.

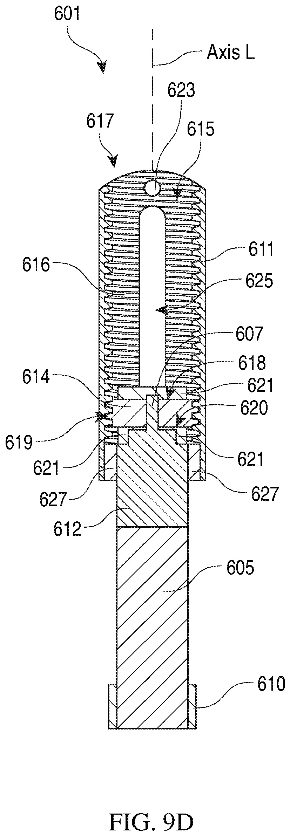

FIGS. 9A-9C are various views of another embodiment of a linear actuator 601 that may be used with the various prosthetic digits described herein. FIG. 9A is a side view of the actuator 601 in an expanded configuration. FIG. 9B is a side view of the actuator 601 in a retracted configuration. FIG. 9C is a cross-sectional view of the actuator 601, as taken along the line 9C-9C indicated in FIG. 9A. FIG. 9D is a cross-section view of the actuator 601 and taken at a ninety degree angle with respect to the line 9C-9C indicated in FIG. 9A. Thus the view in FIG. 9D is rotated ninety degrees with respect to the view shown in FIG. 9C. The actuator 601 may be used in any of the prosthetic digits disclosed herein, such as the prosthetic digits of FIGS. 1A-3H. The actuator 601 may have the same or similar features and/or functions as the actuators 301, 404, 501, and vice versa, except as otherwise described.

As shown in FIGS 9A-9D, the actuator 601 includes a proximal end 613 and extends to a distal end 617. The proximal end 613 may attach to a hand, palm, etc. The distal end 617 may attach to a proximal end of a proximal segment of a prosthetic digit.

Advantageously, the actuator 601 is compact. The actuator 601 is small enough to fit at least partially within a prosthetic digit. The actuator 601 may be sized to fit in a proximal end of a prosthetic digit for a typical sized hand prosthetic. A prosthetic hand may include multiple actuators 601, for example one of the actuators 601 in each of its prosthetic digits. In a closed or retracted configuration, the actuator 601 may have an overall volume of no more than 6,222 mm³. In a closed or retracted configuration, the actuator 601 may have a maximum length of 55 mm and/or a maximum width of 12 mm. In a closed or retracted configuration, the actuator 601 may have an overall volume of no more than 3,000 mm³, no more than 4,000 mm³, no more than 5,000 mm³, no more than 5,500 mm³, no more than 5,750 mm³, no more than 6,000 mm³, no more than 6,500 mm³, no more than 6,750 mm³, or no more than 7,000 mm³. The actuator 601 may have an aspect ratio, defined as the maximum length in a retracted state to a maximum width in a retracted state, of no less than 1.5, no less than 2, no less than 2.5, no less than 3, no less than 3.5, no less than 4, no less than 4.5, or no less than 5.

In an open or extended configuration, in some embodiments the actuator 601 may have an overall volume of no more than 7,918 mm³. In an open or extended configuration, in some embodiments the actuator 601 may have a maximum length of 70 mm and/or a maximum width of 12 mm. In an open or extended configuration, the actuator 601 may have an overall volume of no more than 5,000 mm³, no more than 6,000 mm³, no more than 7,000 mm³, no more than 7,500 mm³, no more than 7,750 mm³, no more than 8,000 mm³, no more than 8,250 mm³, no more than 8,500 mm³, no more than 8,750 mm³, or no more than 9,000 mm³. In an open or extended configuration, the actuator 601 may have a maximum length of no more than 50 mm, no more than 60 mm, no more than 65 mm, no more than 70 mm, no more than 75 mm, no more than 80 mm, no more than 85 mm, or no more than 90 mm. In an open or extended configuration, the actuator 601 may have a maximum width of no more than 6 mm, no more than 8 mm, no more than 10 mm, no more than 11 mm, no more than 12 mm, no more than 13 mm, no more than 14 mm, no more than 16 mm, or no more than 18 mm.

The actuator 601 is a linear actuator that extends and retracts linearly. The actuator 601 produces or results in linear motion. As shown, the actuator 601 includes a motor 605 and a gearbox 612. The motor 605 is in mechanical communication with the gearbox 612. The motor 605 is supplied with power from a battery, which may be in the hand or other location. The actuator 601 includes a shaft 607 extending axially and distally from the gearbox 612 and/or motor 605. The actuator 601 may include a leadscrew 614 coupled to the motor 605 (e.g., coupled to the shaft 607). The leadscrew 614 may be or include a worm gear. The leadscrew 614 may have an external thread 619 that mechanically communicates with internal threads 616 of the housing 611 to cause movement of the housing 611, as described herein.

The actuator 601 includes a thrust bearing 621. The thrust bearing 621 is a rotary rolling-element bearing that permits rotation between parts and is designed to support a predominately axial load. A variety of suitable thrust bearing 621 types may be used, such as thrust ball bearings, cylindrical thrust roller bearings, tapered roller thrust bearings, spherical roller thrust bearings, fluid bearings, magnetic bearings, etc. The thrust bearing 621 may be configured to axially constrain the leadscrew 614 and to take up axial forces generated during actuation of the actuator 601. The thrust bearing 621 may be positioned adjacent a distal end of the gearbox 612. The thrust bearing 621 is fixedly attached to the gearbox 612. The thrust bearing 621 has internal threads on a proximal end thereof (lower end as oriented in the figures) that mechanically communicates with external threads at a distal end of the gearbox 612. The thrust bearing 621 may attach to the gearbox 612 in a variety of other suitable ways, such as pressed, glued, welded, clipped, riveted, reversed male/female thread connection, etc. As shown in FIG. 9C, the thrust bearing 621 includes guide lugs 624 extending proximally from a proximal end thereof. The guide lugs 624 may interact with corresponding distally extending projections from a distal end of the gearbox 612 to prevent rotation of the thrust bearing 621. In some embodiments, the guide lugs 624 may be part of the gearbox 612 and the projections may be part of the thrust bearing 621.

The proximal end of the housing 311 may include an annular stop 627 or other suitable structural feature therein that surrounds and receives the gearbox 612 and motor 605 therethrough. The stop 627 may limit distal axial movement of the housing 611 relative to the gearbox 612. The stop 627 may be a ring, bushing, insert or other feature that contacts the leadscrew 614 at a distal movement limit to prevent farther distal axial travel of the housing 611. The stop 627 may also center and/or stabilize the housing 611 about the gearbox 612 and motor 605, for example during proximal movement and/or positioning of the housing 611 relative to the gearbox 612. In some embodiments, the stop 627 may be a linear or other type of bearing. The stop 627 may be located proximally of the proximal end of the threads 616 of the housing 611, as shown in FIG. 9D.

The leadscrew 614 may be enclosed on top and bottom ends thereof by the thrust bearing 621. For example, the leadscrew 614 may be axially constrained by the thrust bearing 621. The leadscrew 614 may be free to float axially on the motor shaft. The leadscrew 614 is not rotationally constrained by the thrust bearing 621, other than by any frictional forces between the two parts. The thrust bearing 621 contacts the leadscrew 614 at top and bottom bearing interfaces 618, 620, as shown in FIG. 9D. The thrust bearing 621 may be made of a different material than the leadscrew 614, thereby providing a bearing surface while the thrust bearing 621 and the leadscrew 614 are in dynamic contact. The leadscrew 614 may be rotationally constrained during rotational motion by corresponding "D" shaped profiles on the leadscrew 614 bore and motor shaft 607. When the actuator 601 is in motion, an axial force on the leadscrew 614, which results from linear actuation, may be transferred into the thrust bearing 621. In some embodiments, the line of action of the forces during actuation of the actuator 601 include rotation of the shaft 607 transferred to rotation of the leadscrew 614, which is transferred to external threads 619 of the leadscrew 614 to internal threads 616 of the housing 611. The axial force generated by the leadscrew 614, due to axial movement of the housing 611, is transferred to one or the other of the bearing interfaces 618, 620 (depending on the direction of axial movement) and to the corresponding top or bottom surface of the thrust bearing 621, and to the connection with the gearbox 612, which may be a threaded connection as described.

The actuator 601 includes a support 610. The support 610, such as a motor mount or other structure, may carry or otherwise support the actuator 601 and/or the motor 605. The support 610 may be configured to attach the actuator 601 to a hand. For example, the support 610 may include a connector portion 622, such as an opening or a protrusion (e.g., a post), that is compatible with a hand. For example, the support 610 may be configured to receive a pin or other suitable feature in the proximal end thereof to secure, for example rotatably attach, the support 610 with a mount (e.g., a mount on a hand, palm, etc.). As shown in FIG. 9C, the support 610 may include a connector portion622 on the proximal end 613 which may be received in a portion of a mount on a hand, palm, etc., such as a recess or other opening, to secure, for example rotatably attach, the support 610 with said mount. The actuator 601 may rotate about an axis defined by the connector portion 622 as the actuator 601 extends and retracts linearly, as described herein.

The actuator 601 includes the housing 611. The housing 611 extends and retracts axially. The housing 611 defines a cavity 615 therein. The cavity 615 may be a cylindrical opening extending axially inside or through the housing 611. The cavity 615 may have a maximum length and a maximum diameter. The ratio of the maximum length to the maximum diameter may be no less than 1.5, no less than 2, no less than 2.5, no less than 3, no less than 3.5, no less than 4, no less than 4.5, or no less than 5. The ratio of maximum length to maximum diameter may be from 3-6. The cavity 615 may be configured to receive therein a gearbox 612, shaft 607, leadscrew 614, and/or at least a portion of the motor 605. The housing 611 may have an internal thread 616. For example, as shown, the internal thread 616 may be located along the cavity 615 of the housing 611. A proximal end of the housing 611 may be open to the cavity 615. A distal end of the housing 611 may correspond to the distal end 617 of the actuator 601. The distal end of the housing 611 may connect with a proximal segment of a prosthetic digit at a joint. For example, as shown in FIG. 9A, the distal end of the housing 611 may include an opening 623 that is configured to engage a proximal portion of a prosthetic digit. The actuator 601 may rotate about an axis defined by the opening 623 as the actuator 601 extends and retracts linearly, as described herein.

The housing 611 may include a channel 625 along an outer surface of the housing 611. The channel 625 may extend along a portion of the length of the housing 611. The shape of the channel 625 may be a rectangle, oval, or any other suitable shape. The channel 625 may be configured to slidably engage a portion of the thrust bearing 621 and/or leadscrew 614. The channel 625 may guide and/or constrain, e.g. rotationally and/or axially constrain, the thrust bearing 621 within the housing 611. The channel 625 includes proximal and distal surfaces that may axially restrain the thrust bearing 621 at extreme ends of linear actuation. The channel 625 may include side surfaces that rotationally restrain the thrust bearing 621 during actuation. The thrust bearing 611 may include radial projections 626 that extend into the channel 625 and are restrained thereby, as described.

The actuator 601 may include an axially fixed portion and an axially movable portion. The fixed and moveable portions may be respectively fixed and moveable with respect to a longitudinal axis of the actuator 601, such as the axis L shown in FIG. 9C. The axially movable portion of the actuator 601 may slidably engage an outer surface of the fixed portion. The axially movable portion may include the housing 611. The axially fixed portion may include the motor 605, the gearbox 612, the support 610, the leadscrew 614, and the shaft 607.

The actuator 601 may output linear motion to cause rotation of a prosthetic digit. For example, the housing 611 may translate axially to cause rotation of the proximal segment of the prosthetic digit. The motor 605 or other portions of the actuator 601 may use or provide rotary, linear, cyclic and/or other types of motion.

The motor 605 may rotate the leadscrew 614 about the longitudinal Axis L (shown in FIGS. 9C and 9D). The shaft 607 may be configured to be rotated by the motor 605 and be configured to rotate the leadscrew 614 about the longitudinal Axis L in a first rotational direction and a second rotational direction opposite the first rotational direction. The internal thread 616 of the housing 611 may be configured to at least partially engage with the external thread 619 of the leadscrew 614 such that rotation of the leadscrew 614 causes the housing 611 to translate axially along the longitudinal Axis L relative to the leadscrew 614 and/or the support 610 while the leadscrew 614 remains axially stationary. For example, the external thread 619 of the leadscrew 614 may be in mechanical communication with the internal thread 616 of the housing 611. Rotation of the leadscrew 614 in the first rotational direction causes the housing 611 to translate distally relative to the leadscrew 614 and/or the support 610 and rotation of the leadscrew 614 in the second rotational direction causes the housing 611 to translate proximally relative to the leadscrew 614 and/or the support 610.

The actuator 601 may be used in a prosthetic digit having a base configured to attach to a hand, a proximal segment, a middle segment, and a distal segment, an expandable link, and a wheel, such as a worm wheel or other rotatable member. The wheel may be placed in mechanical communication with the actuator 601. The actuator 601 may be configured to cause the wheel to rotate. The prosthetic digit may include a tendon extending distally from the wheel, a pulley rotationally connected to the proximal segment, and a tendon attachment coupled to the middle segment, as described herein, for example with respect to FIGS. 6A-7D. When the actuator 601 is used in a prosthetic digit, translation of the housing 611 in a distal direction along the longitudinal Axis L of the actuator 601 relative to the leadscrew 614 may cause the proximal segment of the prosthetic digit to rotate about a joint, which may cause the middle and distal segments to rotate. Rotation of the wheel by the actuator 601 in a first rotational direction may pull the tendon proximally and cause the distal segment of the prosthetic digit to rotate relative to the middle segment, as described.

The actuator 601 may be used in a variety of other prosthetic digits. The examples provided herein are only some embodiments. The compactness of the actuator 601 allows it to be used in prosthetic hands for each of the prosthetic digits, for example one, two, three, four or five actuators 601 may be used for each of a corresponding prosthetic digit. The actuator 601 may be housed entirely or partially within the prosthetic digit. The actuator 601 may be housed entirely or partially within the hand.

Further, in some embodiments, the actuator 601 may be assembled in a flipped orientation as that described herein. For example, the actuator 601 may be flipped in a proximal to distal direction such that the moveable and fixed portions are reversed. The housing 611 may be axially stationary while the motor 605 and other parts may move axially. The housing 611 may be attached to the prosthetic hand and the motor 605 for instance the motor mount 610 may be attached to the prosthetic digit, for instance the proximal segment. Thus the general principles of the actuator 601 described herein may be used in a variety of contexts that are within the scope of the disclosure.

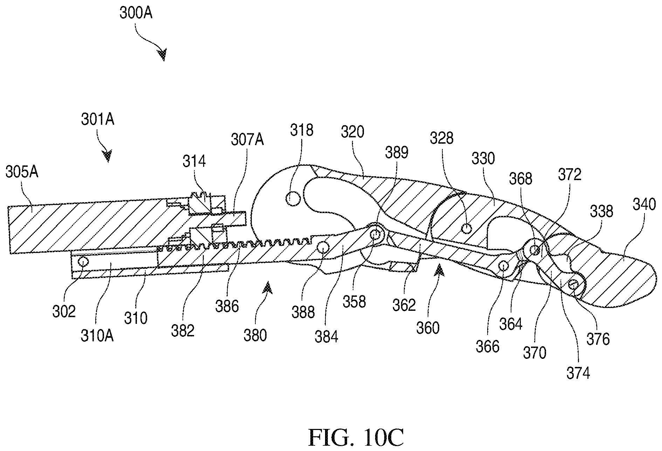

FIGS. 10A-10C are various views of an embodiment of a prosthetic digit 300A. The digit 300A. The digit 300A may be used with the system 100 or hand 200. The digit 300A may have the same or similar features and/or functions as the digit 300, and vice versa, except as otherwise described. FIG. 10A is a perspective view of the digit 300A, FIG. 10B is an exploded view of the digit 300A, and FIG. 10C is a side cross-section view of the digit 300A as taken along the line 10C-10C indicated in FIG. 10A.

The digit 300A includes the segments 320, 330, 340 and links 360, 370, as described herein, for example with respect to FIGS. 3A-3H. The digit 300A further includes an actuator 310A. The actuator 310A includes a motor 305A and shaft 307A, which may have the same or similar features and/or functions as the motor 305 and shaft 307 respectively. The motor 305A rotates the shaft 307A about a longitudinal axis of the motor 305A. There may be a gearbox at a distal end of the motor 305A, as described herein, for example with respect to FIGS. 8A-9C.

The actuator 301A includes a support 310. The motor 305A and gearbox are supported by the support 310. The support 310 extends longitudinally and defines a cavity 310A therein. The cavity and/or sidewall of the support 310 may carry the motor 305A. The shaft 307A extends through openings defined by first and second projections 310B, 310C of the support 310 that extend upwardly therefrom to define a space therebetween. A leadscrew 314 having external threads thereon, as described herein, is attached to the shaft 307A in between the first and second projections 310B, 310C such that rotation of the shaft 307A will rotate the leadscrew 314 in the space defined by the projections 310B, 310C. The leadscrew 314 is axially constrained by the first and second projections 310B, 310C acting as a thrust bearing. In this or any other embodiment of the actuators described herein, the leadscrew may be axially constrained by projections, by the gearbox, by a thrust bearing, and/or by other suitable features. In some embodiments, the leadscrew may not be axially constrained. Thus, any of the features described herein for axially constraining a leadscrew may or may not be applied to any other embodiments. In some embodiments, a nut or endcap may be attached to the distal end of the shaft on the distal side of the second projection 310C to axially secure the motor 305A with the support 310. The support 310 extends from a proximal end having a transverse opening 302 therethrough to a distal end having the projections 310B, 310C extending upwardly therefrom. The cavity 310A extends within the support 310 from the proximal end to the distal end.

The actuator 310A includes a rack 380. The rack 380 may be a worm rack. The rack 380 extends from a proximal end 382 to a distal end 384. The proximal end 382 includes an elongated section having threads 386. The threads 386 may be partial threads as shown. The threads 386 extend transversely and ae located along the length of the rack 380. The distal end 384 includes an opening 389 that is configured to connect with the connection 358 at the proximal end 362 of the proximal link 360. The rack 380 may be a section of the inner threaded portion of the housing 311 described herein. The rack 380 may include a joint 388 that is attached to the proximal digit segment 320, and about which the rack 380 may rotate as the rack 380 pushes and pulls at the joint 388 during axial movement. The joint 388 may be an opening having a pin extending therethrough to rotationally connect the rack 380 and the proximal segment 320. The rack 380, by pushing or pulling at the joint 388, may cause the proximal segment 320 to rotate or pivot about the joint 318.

The rack 380 may be a portion of the housing 311, which is described herein for example with respect to FIGS. 3D-3H. The rack 380 may be a lower proximal portion of the housing 311. The rack 380 may slide linearly within the cavity 310A of the support 310. The rack 380 may translate axially due to engagement of the threads 386 with the threads of the leadscrew 314. As the leadscrew 314 rotates, the threads of the leadscrew engage the threads 386 of the rack 380 to cause the rack 380 to move axially. The rack 380 may translate distally in response to rotation of the leadscrew in a first rotational direction, and the rack 380 may translate proximally in response to rotation of the leadscrew in a second rotational direction that is opposite to the first rotational direction. The support 310 may act as a linear bearing guideway for the rack 380. In some embodiments, the support 310 may include a "key"-like cross-section to locate and guide the rack 380 during axial movement.

Axial movement of the rack 380 will cause the proximal end 362 of the proximal link 360 to correspondingly move axially. Axial translation of the proximal link 360 will cause the digit 300A to rotate closed or open depending on the direction of axial movement of the link 360, as described herein, for example with respect to FIGS. 3F-3H. The proximal segment 320 may rotate about the joint 318 as the rack 380 pushes and pulls at the joint 388 to cause the distal end 384 to push or pull at the connection 358 via a pin through the opening 389.

The digit 300A may include a housing or cover over the actuator 301A and/or other portions of the digit 300A. In some embodiments, the actuator 301A and/or other features of the digit 300A may be located within a prosthetic hand, such as the palm region. In some embodiments, the actuator 301 may rotate slightly about a transverse axis to accommodate rotation of the digit 300A, for example at the opening 302 of the support 310, which may be located inside the hand or palm.

The digit 300A with the linearly translatable rack 380 may improve performance and extend the life of the digit 300A, for example by reducing the contact area and thus the friction between the threads of the lead screw 314 and the rack 380.

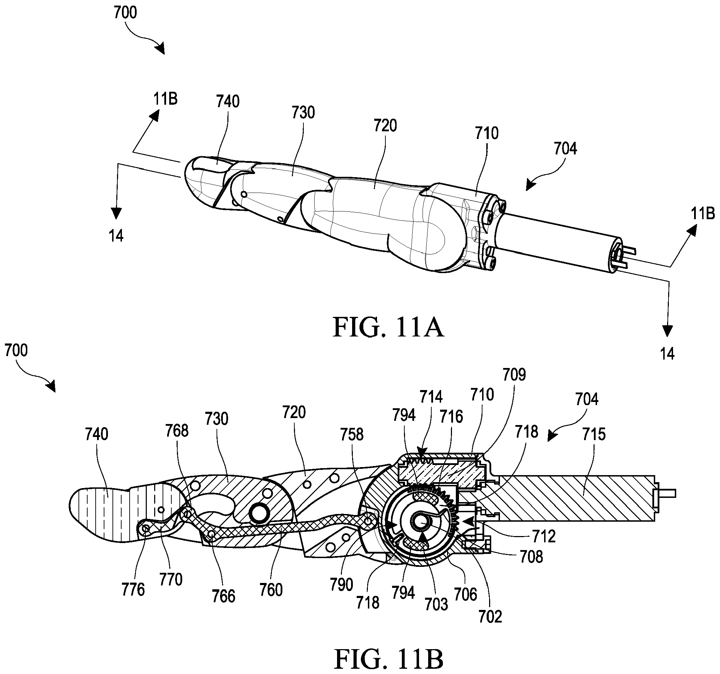

FIGS. 11A-11B are various views of another embodiment of a prosthetic digit 700. The digit 700 may be used with the system 100 or hand 200. The digit 700 includes a mount 710, a proximal segment 720, a middle segment 730, and a distal segment 740. The mount 710 and segments 720, 730, 740 may have the same or similar features and/or functions as respectively the mounts 350, 410 and segments 320, 330, 340, 420, 430, 440, and thus may articulate, for example rotate, relative to each other, etc.

The digit 700 includes mechanically-connected rigid links, including a proximal link 760 and a distal link 770. The links 760, 770 may have the same or similar features and/or functions as the links 360, 370. For example, the mount 710 may be rotatably attached to the proximal end of the proximal link 760 about a connection 758. The proximal link 760 is rotatably attached to the middle segment 730 of the digit 700 about a pivot 766. The proximal link 760 may include a dogleg, where the proximal end of the proximal link 760 extends along a first axis and the distal end of the proximal link extends along a second axis that is at an angle relative to the first axis. The pivot 766 may be located at or near the vertex of the dogleg of the proximal link 760. The distal end of the proximal link 760 is rotatably attached to the proximal end of the distal link 770 about a connection 768. The distal end of the distal link 770 is rotatably attached to the distal segment 740 of the digit 700 about a pivot 776.

The digit 700 includes an actuator 704, which may have the same or similar features and/or functions as the actuators 301, 404, except as otherwise described. For example, the actuator 704 may include a motor 715 supplied with power from a battery, which may be in the hand or other location. The motor 715 may have an output shaft that extends, for example distally, therefrom, and that mechanically communicates with an off-axis shaft 709.

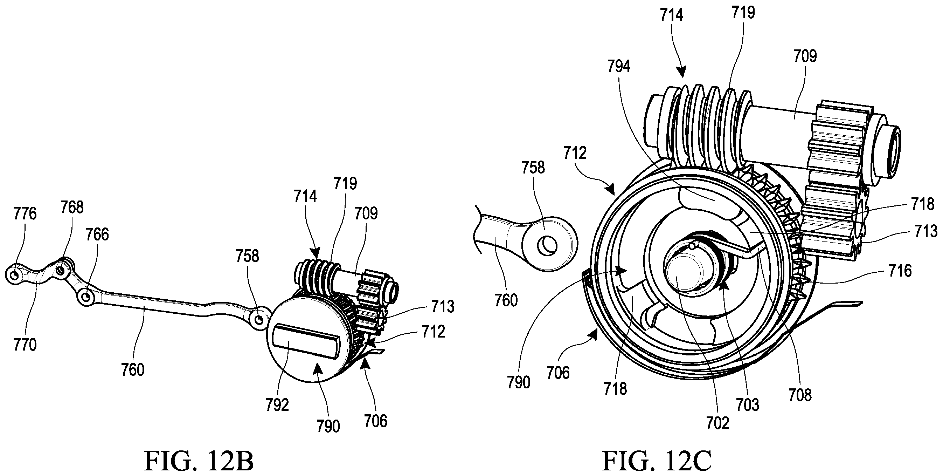

The actuator 704 includes a worm wheel 712 and a worm gear 714, which may have the same or similar features and/or functions as respectively the worm wheel and worm gear 412, 414, except as otherwise described. For example, the worm gear 714 having external threads 719 thereon may be in mechanical communication with the shaft 709. Actuation of the motor 715 causes motion to be transmitted via a pinion gear 713 (see FIGS. 12B and 12C) to the shaft 709 to rotate the worm gear 714. The worm wheel 712 may have external teeth 716 thereon. In some embodiments, only a portion of the outer circumference of the worm wheel 712 includes external teeth 716 (e.g., the portion of the outer circumference of the worm wheel 712 positioned adjacent to the worm gear 714). The remainder of the outer circumference of the worm wheel 712 may be smooth or otherwise not have teeth. This configuration can advantageously allow for a compact worm wheel 712 and worm gear 714 system. The threads 719 (see FIGS, 12B and 12C) of the worm gear 714 contact the teeth 716 of the worm wheel 712 to cause rotational motion of the worm wheel 712. The worm wheel 712 may be rotated a first rotational direction to cause a first rotation of the digit 700 in a first direction (e.g. to close the digit 700). The worm wheel 712 may be rotated in a second rotational direction that is opposite the first rotational direction to allow for a second rotation of the digit 700 in a second direction that is opposite the first direction (e.g. to open the digit).

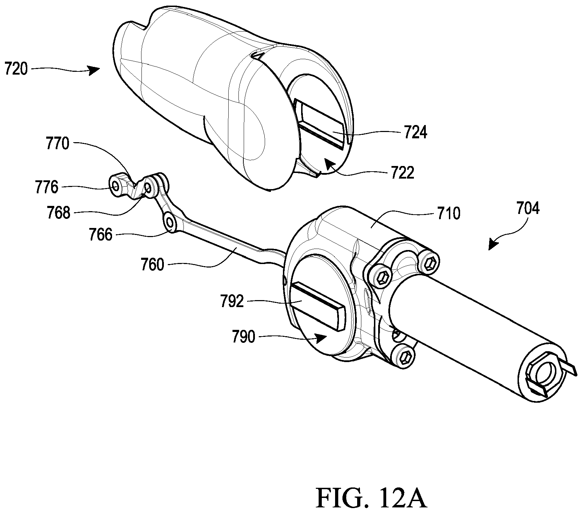

FIGS. 12A-12C are various views of the actuator 704 of the digit 700. FIG. 12A is a partial exploded view of the actuator 704, and FIGS. 12B and 12C show the actuator 704 with various features removed or hidden for clarity. The actuator 704 of the digit 700 may comprise a central axle 790 having a drive key 792 configured to engage a portion of the proximal segment 720 of the digit 700. For example, in some embodiments, the drive key 792 is positioned on an outer surface of the central axle 790 and has an extended length and width protruding outwardly from the outer surface of the central axle 790. An inner surface 722 of the proximal segment 720 of the digit 700 may comprise a mating feature 724, such as a recess, opening, and/or groove, with a shape that corresponds with the shape of the drive key 792 of the central axle 790. The mating feature 724 of the proximal segment 720 may receive the drive key 792 of the central axle 790 therein to transmit a rotational force from the central axle 790 to the proximal segment 720. In some embodiments, the ratio of the rotational angle of the drive key 792 to the rotational angle of the proximal segment 720 is 1:1.

In some embodiments, the central axle 790 includes a first drive key 792 protruding outwardly in a first direction from a first outer surface of the central axle 790 and a second drive key 792 protruding outwardly from a second outer surface of the central axle 790 in a second direction that is opposite the first direction. The proximal segment 720 may include a first inner surface 722 with a first mating feature 724 for receiving the first drive key 792 and a second inner surface 722 with a second mating feature 724 for receiving the second drive key 792.

In some embodiments, the central axle 790 may include one or more drive tabs 794. The drive tabs 794 may each have an extended, arcuate length and width protruding axially from an inner surface of the central axle 790. In some embodiments, the central axle 790 includes a first drive tab 794 and a second drive tab 794 positioned radially opposite the first drive tab 794.

In some embodiments, the worm wheel 712 may include one or more corresponding drive tabs 718. For example, the worm wheel 712 may include a first drive tab 718 and a second drive tab 718 positioned radially opposite the first drive tab 718. The drive tabs 718 of the worm wheel 712 may extend radially inward from an inner surface of the worm wheel 712 toward a central axis of the worm wheel 712. The drive tabs 718 of the worm wheel 712 may be positioned between the first and second drive tabs 794 of the central axle 790. In some embodiments, one or more of the drive tabs 794 of the central axle 790 engages one or more of the drive tabs 718 of the worm wheel 712 (e.g., contacts, abuts, connects to, etc.) to transmit a rotational force of the worm wheel 712 to the central axle 790.

The drive mechanism of the digit 700 may include a spring 703 (e.g., a torsion spring). The spring 703 may be coupled to (e.g., circumferentially surround) an axially extending member 702 that extends axially along the central axis of the worm wheel 712 and/or central axle 790. The spring 703 may be configured to rotationally bias the worm wheel 712 in an angular direction to maintain the relative positions of the central axle 790 and the worm wheel 712. For example, the spring 703 may include a flange 708 that extends further radially outward than the rest of the spring 703. The flange 708 may engage one of the drive tabs 718 of the worm wheel 712. For example, in some embodiments, the worm wheel 712 and the central axle 790 are positioned such that one of the drive tabs 794 of the central axle 790 abuts a first surface of one of the drive tabs 718 of the worm wheel 712 and the flange 708 abuts a second surface of the drive tab 718 opposite the first surface of the drive tab 718. This configuration enables the rotational force of the worm wheel 712 to be transmitted to the central axle 790 while maintaining the relative positions of the worm wheel 712 and the central axle 790. This configuration also allows the digit 700 to be closed independent of the drive mechanism of the digit 700, as further described below.

In some embodiments, the digit 700 may be opened and/or closed with or without utilizing the actuator 704. For example, the digit 700 can have a worm wheel driven movement mode (e.g., driven by the actuator 704) and a manual movement mode (e.g., driven by an external force). When the digit 700 is in an open position, application of an external force on the digit 700 in a closing direction may cause the digit 700 to fold to a closed position. In some embodiments, in the manual movement mode, unlike in the worm wheel driven movement mode, the actuator 704 does not drive the worm wheel 712. For example, in the manual movement mode, the actuator 704 and the worm wheel 712 remain stationary. In the manual movement mode, the central axle 790 rotates in response to the application of an external force to the digit 700 while the worm wheel 712 remains stationary because the spring flange 708 allows for rotational movement when its spring biasing force is overcome. The rotation of the central axle 790 may cause the segments 720, 730, 740 of the digit 700 to rotate to a closed position. In the manual movement mode, the projections 718 of the worm wheel 712 may limit the range of rotation of one or more of the drive tabs 794 of the central axle 790 and therefore the range of rotation of the central axle 790. The spring 703 may rotate and store energy due to the manual movement of the digit 700 to the closed position due to the application of an external force to the digit 700. In some embodiments, when the external force is removed from the digit 700, the spring 703 may use the stored potential energy to rotate and cause the digit 700 to return to the open position.

The manual movement mode of the digit 700 can advantageously serve as a mechanical protection system when external forces act on the digit 700, such as when a user falls on the digit 700 or applies pressure to the digit 700 to get up from a chair, etc. The manual closure of the digit 700 may allow the external load to be supported by components of the digit 700 other than the drive mechanism (e.g., gearbox). This can prevent damage that may otherwise have been caused to the drive mechanism of the digit 700.

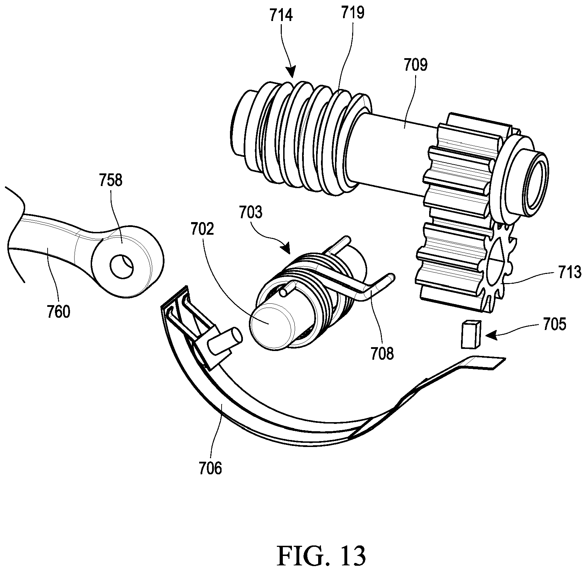

FIG. 13 illustrates the positions of encoders 705, 706 within the digit 700. In some embodiments, the digit 700 includes a plurality of encoders 705, 706 mounted to the gearbox. For example, in some embodiments, the digit 700 includes a first type of encoder for the worm wheel driven movement mode and a second type of encoder for the manual movement mode. As shown, the digit 700 may include a potentiometer strip encoder 706 and a magnetometer encoder 705. The potentiometer strip encoder 706 may be coupled to the worm wheel 712. The magnetometer encoder 705 may be positioned between the potentiometer strip encoder 706 and the pinion gear 713. The potentiometer strip encoder 706 may measure the position of the digit 700 by measuring the absolute position of the motor drive. The magnetometer encoder 705 may be an absolute magnetic hall effect encoder. The magnetometer encoder 705 may measure the position of the digit 700 by measuring the degree of rotation of a diametrically magnetized axial magnet disposed within the axially extending member 702 at the center of the central axle 790.

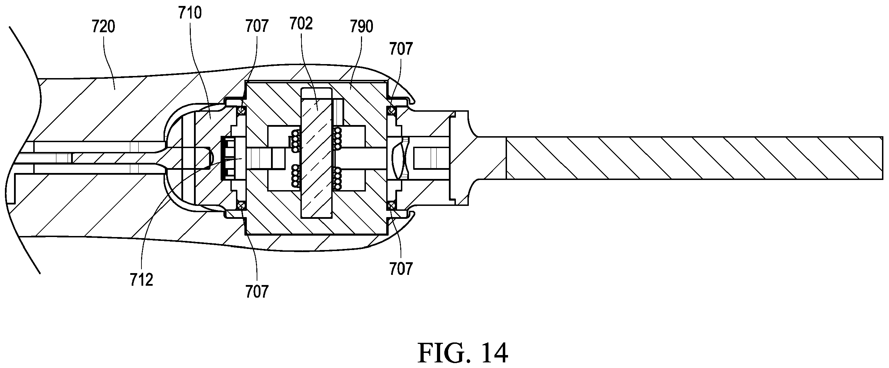

FIG. 14 is a cross-sectional view of a portion of the digit 700 illustrating waterproof seals 707 within the digit 700. In some embodiments, the digit 700 may be waterproof (e.g., rated IP68). The digit 700 may include seals 707, such as O-ring seals, lip seals, and/or other dynamic seals, to seal the components within the central axle 790 from water ingress. For example, the seals 707 may be positioned in gaps between the central axle 790 and the mount 710.

In some embodiments, the digit 700 may include any of the various embodiments of the digits and actuators described herein, or features thereof. The digit 700 may include the compact actuator 501 or 601, or features thereof. For example, the digit 700 may be modified to include the motor 505 or motor 605 in place of the motor 715. Other suitable substitutions or modifications to the digit 700 using any of the prosthetic features described herein may be implemented in a variety of embodiments.

Various modifications to the implementations described in this disclosure can be readily apparent to those skilled in the art, and the generic principles defined herein can be applied to other implementations without departing from the scope of the claims. Thus, the disclosure is not intended to be limited to the implementations shown herein, but is to be accorded the widest scope consistent with the claims, the principles and the novel features disclosed herein. The word "example" is used exclusively herein to mean "serving as an example, instance, or illustration." Any implementation described herein as "example" is not necessarily to be construed as preferred or advantageous over other implementations.

Certain features that are described in this specification in the context of separate implementations also can be implemented in combination in a single implementation. Conversely, various features that are described in the context of a single implementation also can be implemented in multiple implementations separately or in any suitable sub-combination. Moreover, although features can be described above as acting in certain combinations and even initially claimed as such, one or more features from a claimed combination can in some cases be excised from the combination, and the claimed combination can be directed to a sub-combination or variation of a sub-combination.

Similarly, while operations are depicted in the drawings in a particular order, this should not be understood as requiring that such operations be performed in the particular order shown or in sequential order, or that all illustrated operations be performed, to achieve desirable results. In certain circumstances, multitasking and parallel processing can be advantageous. Moreover, the separation of various system components in the implementations described above should not be understood as requiring such separation in all implementations, and it should be understood that the described program components and systems can generally be integrated together in a single software product or packaged into multiple software products. Additionally, other implementations are within the scope of the following claims. In some cases, the actions recited in the claims can be performed in a different order and still achieve desirable results.

It will be understood by those within the art that, in general, terms used herein are generally intended as "open" terms (e.g., the term "including" should be interpreted as "including but not limited to," the term "having" should be interpreted as "having at least," the term "includes" should be interpreted as "includes but is not limited to," etc.). It will be further understood by those within the art that if a specific number of an introduced claim recitation is intended, such an intent will be explicitly recited in the claim, and in the absence of such recitation no such intent is present. For example, as an aid to understanding, the following appended claims may contain usage of the introductory phrases "at least one" and "one or more" to introduce claim recitations. However, the use of such phrases should not be construed to imply that the introduction of a claim recitation by the indefinite articles "a" or "an" limits any particular claim containing such introduced claim recitation to embodiments containing only one such recitation, even when the same claim includes the introductory phrases "one or more" or "at least one" and indefinite articles such as "a" or "an" (e.g., "a" and/or "an" should typically be interpreted to mean "at least one" or "one or more"); the same holds true for the use of definite articles used to introduce claim recitations. In addition, even if a specific number of an introduced claim recitation is explicitly recited, those skilled in the art will recognize that such recitation should typically be interpreted to mean *at least* the recited number (e.g., the bare recitation of "two recitations," without other modifiers, typically means *at least* two recitations, or *two or more* recitations). Furthermore, in those instances where a convention analogous to "at least one of A, B, and C, etc." is used, in general such a construction is intended in the sense one having skill in the art would understand the convention (e.g., "a system having at least one of A, B, and C" would include but not be limited to systems that have A alone, B alone, C alone, A and B together, A and C together, B and C together, and/or A, B, and C together, etc.). In those instances where a convention analogous to "at least one of A, B, or C, etc." is used, in general such a construction is intended in the sense one having skill in the art would understand the convention (e.g., "a system having at least one of A, B, or C" would include but not be limited to systems that have A alone, B alone, C alone, A and B together, A and C together, B and C together, and/or A, B, and C together, etc.). It will be further understood by those within the art that virtually any disjunctive word and/or phrase presenting two or more alternative terms, whether in the description, claims, or drawings, should be understood to contemplate the possibilities of including one of the terms, either of the terms, or both terms. For example, the phrase "A or B" will be understood to include the possibilities of "A" or "B" or "A and B."

## Claims

1. An actuator (301, 404, 501, 601, 704) for a prosthetic digit (120, 220, 300, 400, 700), the actuator comprising:
a gearbox (512, 612) and a motor (305, 405, 505, 605, 715) in mechanical communication with the gearbox (512, 612);
a shaft (307, 409, 507, 607, 709) extending axially and distally from the gearbox (512, 612);
a leadscrew (314, 514, 614) coupled to the shaft (307, 409, 507, 607, 709), the leadscrew (314, 514, 614) having an external thread, wherein the motor (305, 405, 505, 605, 715) is configured to cause the leadscrew (314, 514, 614) to rotate in a first rotational direction; and a housing (311, 403, 459, 511, 611) or other member configured to couple with a prosthetic digit (120, 220, 300, 400, 700), the housing (311, 403, 459, 511, 611) or other member configured to engage the external thread of the leadscrew (314, 514, 614), **characterized in that** rotation of the leadscrew (314, 514, 614) causes the housing or other member to translate axially relative to the leadscrew (314, 514, 614) to thereby cause a proximal digit segment (320) of the prosthetic digit (120, 220, 300, 400, 700) to rotate about a pivot and thus the prosthetic digit (120, 220, 300, 400, 700) to articulate.

2. The actuator (301, 404, 501, 601, 704) of Claim 1, wherein the housing (311, 403, 459, 511, 611) or other member defines an internal cavity configured to receive therein the gearbox (512, 612), shaft (307, 409, 507, 607, 709), leadscrew (314, 514, 614), and at least part of the motor (305, 405, 505, 605, 715).

3. The actuator (301, 404, 501, 601, 704) of any one of Claims 1 and 2, wherein the leadscrew (314, 514, 614) is configured to remain axially stationary relative to the motor (305, 405, 505, 605, 715).

4. The actuator (301, 404, 501, 601, 704) of any one of Claims 1-3, wherein rotation of the leadscrew (314, 514, 614) in the first rotational direction causes the housing (311, 403, 459, 511, 611) or other member to translate distally relative to the leadscrew (314, 514, 614) and rotation of the leadscrew (314, 514, 614) in a second rotational direction opposite the first rotational direction causes the housing (311, 403, 459, 511, 611) or other member to translate proximally relative to the leadscrew (314, 514, 614).

5. A prosthetic digit (120, 220, 300, 400, 700) comprising:
an actuator (301, 404, 501, 601, 704) comprising:
a mount configured to attach to a hand;
a motor (305, 405, 505, 605, 715) supported by the mount; a leadscrew (314, 514, 614) coupled with the motor (305, 405, 505, 605, 715), the leadscrew (314, 514, 614) having an external thread, wherein the motor (305, 405, 505, 605, 715) is configured to cause the leadscrew (314, 514, 614) to rotate about a first axis; and
a housing (311, 403, 459, 511, 611) or other member extending along the first axis and configured to couple with a proximal end of a prosthetic digit (120, 220, 300, 400, 700), the housing (311, 403, 459, 511, 611) or other member engaged with the external thread of the leadscrew (314, 514, 614), **characterized in that** the leadscrew (314, 514, 614) is configured to remain axially stationary relative to the motor (305, 405, 505, 605, 715) and **in that** rotation of the leadscrew (314, 514, 614) causes
the housing (311, 403, 459, 511, 611) or other member to translate along the first axis to thereby cause the prosthetic digit (120, 220, 300, 400, 700) to rotate.

6. The prosthetic digit (120, 220, 300, 400, 700) of Claim 5, wherein a proximal segment (320) of the prosthetic digit (120, 220, 300, 400, 700) is configured to rotate about a first pivot in response to the housing (311, 403, 459, 511, 611) or other member translating distally along the first axis relative to the leadscrew (314, 514, 614).

7. The prosthetic digit (120, 220, 300, 400, 700) of any one of Claims 5 and 6, wherein the internal cavity of the housing (311, 403, 459, 511, 611) or other member is configured to receive therein the leadscrew (314, 514, 614) and at least part of the motor (305, 405, 505, 605, 715).

8. The prosthetic digit (120, 220, 300, 400, 700) of any one of Claims 5-7, wherein the actuator (301, 404, 501, 601, 704) further comprises a thrust bearing configured to axially restrain the leadscrew (314, 514, 614).

9. The prosthetic digit (120, 220, 300, 400, 700) of any one of Claims 5-8, wherein rotation of the leadscrew (314, 514, 614) in a first rotational direction causes the housing (311, 403, 459, 511, 611) or other member to translate distally relative to the leadscrew (314, 514, 614) and rotation of the leadscrew (314, 514, 614) in a second rotational direction opposite the first rotational direction causes the housing (311, 403, 459, 511, 611) or other member to translate proximally relative to the leadscrew (314, 514,614).

10. The actuator (301) of any one of Claims 1-4, wherein the housing (311) or other member comprises a rack (380).

11. The actuator (301) of Claim 10, wherein the rack (380) includes an elongated portion having partial threads (386), and wherein the partial threads (386) extend transversely along the length of the rack (380).

12. The actuator (301) of any one of Claims 10 and 11, wherein the rack (380) comprises a joint (388), the joint (388) being attached to the proximal digit segment (320).

13. The prosthetic digit (300) of any one of Claims 5-9, wherein the housing (311) or other member comprises a rack (380).

14. The prosthetic digit (300) of Claim 13, wherein the rack (380) is a worm rack.

15. The prosthetic digit (300) of any one of Claims 13 and 14, wherein the rack (380) includes an elongated portion having threads (386), and wherein the threads extend transversely along the length of the rack (380).

## Patentansprüche

1. Ein Stellglied (301, 404, 501, 601, 704) für eine Fingerprothese (120, 220, 300, 400, 700), wobei das Stellglied Folgendes umfasst:
ein Getriebe (512, 612) und einen Motor (305, 405, 505, 605, 715), der in mechanischer Verbindung zum Getriebe (512, 612) steht,
eine Welle (307, 409, 507, 607, 709), die sich axial und distal vom Getriebe (512, 612) erstreckt,
eine Gewindespindel (314, 514, 614), die mit der Welle (307, 409, 507, 607, 709) verbunden ist, wobei die Gewindespindel (314, 514, 614) ein Außengewinde aufweist und der Motor (305, 405, 505, 605, 715) so ausgelegt ist, dass er die Gewindespindel (314, 514, 614) zum Drehen in einer ersten Drehrichtung bringt, und
ein Gehäuse (311, 403, 459, 511, 611) oder ein anderes Organ, das zur Verbindung mit einer Fingerprothese (120, 220, 300, 400, 700) ausgelegt ist, wobei das Gehäuse (311, 403, 459, 511, 611) oder das andere Organ zum Eingreifen in das Außengewinde der Gewindespindel (314, 514, 614) ausgelegt ist, **dadurch gekennzeichnet, dass**
die Drehung der Gewindespindel (314, 514, 614) das Gehäuse oder das andere Organ zu einer axialen Querbewegung in Bezug auf die Gewindespindel (314, 514, 614) bringt und dadurch ein proximales Fingersegment (320) der Fingerprothese (120, 220, 300, 400, 700) zur Drehung um ein Drehgelenk bringt und somit die Fingerprothese (120, 220, 300, 400, 700) gelenkig macht.

2. Das Stellglied (301, 404, 501, 601, 704) gemäß Anspruch 1, wobei das Gehäuse (311, 403, 459, 511, 611) oder das andere Organ einen inneren Hohlraum ausbildet, der zur Aufnahme von Getriebe (512, 612), Welle (307, 409, 507, 607, 709), Gewindespindel (314, 514, 614) und zumindest eines Teils des Motors (305, 405, 505, 605, 715) dient.

3. Das Stellglied (301, 404, 501, 601, 704) gemäß einem der Ansprüche 1 und 2, wobei die Gewindespindel (314, 514, 614) so ausgelegt ist, dass sie axial stationär in Bezug auf den Motor (305, 405, 505, 605, 715) bleibt.

4. Das Stellglied (301, 404, 501, 601, 704) gemäß einem der Ansprüche 1 bis 3, wobei die Drehung der Gewindespindel (314, 514, 614) in einer ersten Drehrichtung das Gehäuse (311, 403, 459, 511, 611) oder andere Organ zur distalen Querbewegung in Bezug auf die Gewindespindel (314, 514, 614) bringt und in einer zweiten, zur ersten entgegengesetzten Drehrichtung der Gewindespindel (314, 514, 614) das Gehäuse (311, 403, 459, 511, 611) oder andere Organ zur proximalen Querbewegung in Bezug auf die Gewindespindel (314, 514, 614) bringt.

5. Eine Fingerprothese (120, 200, 200, 400 700) mit
einem Stellglied (301, 404, 501, 601, 704) mit:
einer zur Befestigung an einer Hand vorgesehenen Halterung,
einem von der Halterung gestützten Motor (305, 405, 505, 605, 715),
einer Gewindespindel (314, 514, 614), die mit dem Motor (305, 405, 505, 605, 715) verbunden ist, wobei die Gewindespindel (314, 514, 614) ein Außengewinde aufweist und der Motor (305, 405, 505, 605, 715) so ausgelegt ist, dass er die Gewindespindel (314, 514, 614) zur Drehung um eine erste Achse bringt, und
ein Gehäuse (311, 403, 459, 511, 611) oder ein anderes Organ, das sich entlang der ersten Achse erstreckt und zur Verbindung mit einem proximalen Ende einer Fingerprothese (120, 220, 300, 400, 700) ausgelegt ist, wobei das Gehäuse (311, 403, 459, 511, 611) oder das andere Organ zum Eingreifen in das Außengewinde der Gewindespindel (314, 514, 614) ausgelegt ist, **dadurch gekennzeichnet, dass** die Gewindespindel (314, 514, 614) so ausgelegt ist, dass sie axial stationär zum Motor (305, 405, 505, 605, 715) bleibt und die Drehung der Gewindespindel (314, 514, 614) das Gehäuse (311, 403, 459, 511, 611) oder andere Organ zur Querbewegung entlang der ersten Achse bringt und dadurch die Drehung der Fingerprothese (120, 200, 200, 400 700) bewirkt.

6. Die Fingerprothese (120, 200, 200, 400 700) gemäß Anspruch 5, wobei ein proximales Segment (320) der Fingerprothese (120, 200, 200, 400 700) so ausgelegt ist, dass es sich als Antwort auf die distale Querbewegung des Gehäuses (311, 403, 459, 511, 611) oder anderen Organs entlang der ersten Achse in Bezug auf die Gewindespindel (314, 514, 614) um ein erstes Drehgelenk dreht.

7. Die Fingerprothese (120, 200, 200, 400 700) gemäß Anspruch 5 oder 6, wobei der innere Hohlraum des Gehäuses (311, 403, 459, 511, 611) oder anderen Organs zur Aufnahme von Gewindespindel (314, 514, 614) und mindestens einem Teil des Motors (305, 405, 505,605, 715) ausgelegt ist.

8. Die Fingerprothese (120, 200, 200, 400 700) gemäß einem der Ansprüche 5-7, wobei das Stellglied (301, 404, 501, 601, 704) weiterhin ein Drucklager aufweist, das für den axialen Rückhalt der Gewindespindel (314, 514, 614) ausgelegt ist.

9. Die Fingerprothese (120, 200, 200, 400 700) gemäß einem der Ansprüche 5-8, wobei die Drehung der Gewindespindel (314, 514, 614) in einer ersten Drehrichtung das Gehäuse (311, 403, 459, 511, 611) oder andere Organ zur distalen Querbewegung in Bezug auf die Gewindespindel (314, 514, 614) bringt und in einer zweiten, zur ersten entgegengesetzten Drehrichtung der Gewindespindel (314, 514, 614) das Gehäuse (311, 403, 459, 511, 611) oder andere Organ zur proximalen Querbewegung in Bezug auf die Gewindespindel (314, 514, 614) bringt.

10. Das Stellglied (301) gemäß einem der Ansprüche 1-4, wobei das Gehäuse (311) oder andere Organ eine Zahnstange (380) umfasst.

11. Das Stellglied (301) gemäß Anspruch 10, wobei die Zahnstange (380) einen länglichen Bereich mit Teilgewinden (386) umfasst und die Teilgewinde (386) sich in Querrichtung entlang der Länge der Zahnstange (380) erstrecken.

12. Das Stellglied (301) gemäß einem der Ansprüche 10 und 11, wobei die Zahnstange (380) eine Dichtung (388) aufweist und die Dichtung (388) an dem proximalen Fingersegment (320) befestigt ist.

13. Die Fingerprothese (300) gemäß einem der Ansprüche 5-9, wobei das Gehäuse (311) oder andere Organ eine Zahnstange (380) umfasst.

14. Die Fingerprothese (300) gemäß Anspruch 13, wobei die Zahnstange ein Schneckengetriebe ist.

15. Die Fingerprothese (300) gemäß einem der Ansprüche 13 und 14, wobei die Zahnstange (380) einen länglichen Bereich mit Gewinden (386) umfasst und die Gewinde sich in Querrichtung entlang der Länge der Zahnstange (380) erstrecken.

## Revendications

1. Actionneur (301, 404, 501, 601, 704) pour une prothèse de doigt (120, 220, 300, 400, 700), l'actionneur comprenant :
une boîte d'engrenage (512, 612) et un moteur électrique (305, 405, 505, 605, 715) en communication mécanique avec la boîte d'engrenage (512, 612) ;
un arbre (307, 409, 507, 607, 709) s'étendant axialement et distalement depuis la boîte d'engrenage (512, 612) ;
une vis sans fin (314, 514, 614) couplée à l'arbre (307, 409, 507, 607, 709), la vis sans fin (314, 514, 614) présentant un filetage externe, où le moteur électrique (305, 405, 505, 605, 715) est configuré pour amener la vis sans fin (314, 514, 614) à tourner selon un premier sens de rotation ; et
un logement (311, 403, 459, 511, 611) ou un autre élément configuré pour se coupler avec une prothèse de doigt (120, 220, 300, 400, 700), le logement (311, 403, 459, 511, 611) ou l'autre élément étant configuré pour engager le filetage externe de la vis sans fin (314, 514, 614), **caractérisé en ce qu'**une rotation de la vis sans fin (314, 514, 614) amène le logement ou l'autre élément à réaliser une translation de manière axiale par rapport à la vis sans fin (314, 514, 614) afin d'amener ainsi un segment de doigt proximal (320) de la prothèse de doigt (120, 220, 300, 400, 700) à tourner autour d'un pivot et d'amener ainsi la prothèse de doigt (120, 220, 300, 400, 700) à s'articuler.

2. Actionneur (301, 404, 501, 601, 704) selon la revendication 1, dans lequel le logement (311, 403, 459, 511, 611) ou l'autre élément définit une cavité interne configurée pour recevoir en son sein la boîte d'engrenage (512, 612), l'arbre (307, 409, 507, 607, 709), la vis sans fin (314, 514, 614), et au moins une partie du moteur électrique (305, 405, 505, 605, 715).

3. Actionneur (301, 404, 501, 601, 704) selon l'une quelconque des revendications 1 et 2, dans lequel la vis sans fin (314, 514, 614) est configurée pour demeurer axialement stationnaire par rapport au moteur électrique (305, 405, 505, 605, 715).

4. Actionneur (301, 404, 501, 601, 704) selon l'une quelconque des revendications 1 à 3, dans lequel une rotation de la vis sans fin (314, 514, 614) selon le premier sens de rotation amène le logement (311, 403, 459, 511, 611) ou l'autre élément à réaliser une translation de manière distale par rapport à la vis sans fin (314, 514, 614) et une rotation de la vis sans fin (314, 514, 614) selon un second sens de rotation opposé au premier sens de rotation amène le logement (311, 403, 459, 511, 611) ou l'autre élément à réaliser une translation de manière proximale par rapport à la vis sans fin (314, 514, 614).

5. Prothèse de doigt (120, 220, 300, 400, 700) comprenant :
un actionneur (301, 404, 501, 601, 704) comprenant :
un support configuré pour être attaché sur une main ;
un moteur électrique (305, 405, 505, 605, 715) supporté par le support ;
une vis sans fin (314, 514, 614) couplée au moteur électrique (305, 405, 505, 605, 715), la vis sans fin (314, 514, 614) présentant un filetage externe, où le moteur électrique (305, 405, 505, 605, 715) est configuré pour amener la vis sans fin (314, 514, 614) à tourner autour d'un premier axe ;
un logement (311, 403, 459, 511, 611) ou un autre élément s'étendant le long du premier axe et configuré pour se coupler avec une extrémité proximale d'une prothèse de doigt (120, 220, 300, 400, 700), le logement (311, 403, 459, 511, 611) ou l'autre élément étant engagé avec le filetage externe de la vis sans fin (314, 514, 614), **caractérisé en ce que** la vis sans fin (314, 514, 614) est configurée pour demeurer axialement stationnaire par rapport au moteur électrique (305, 405, 505, 605, 715) et **en ce qu'**une rotation de la vis sans fin (314, 514, 614) amène le logement (311, 403, 459, 511, 611) ou l'autre élément à réaliser une translation le long du premier axe afin d'amener ainsi la prothèse de doigt (120, 220, 300, 400, 700) à tourner.

6. Prothèse de doigt (120, 220, 300, 400, 700) selon la revendication 5, dans laquelle un segment proximal (320) de la prothèse de doigt (120, 220, 300, 400, 700) est configuré pour tourner autour d'un premier pivot en réponse à la translation du logement (311, 403, 459, 511, 611) ou de l'autre élément de manière distale le long du premier axe par rapport à la vis sans fin (314, 514, 614).

7. Prothèse de doigt (120, 220, 300, 400, 700) selon l'une quelconque des revendications 5 et 6, dans laquelle la cavité interne du logement (311, 403, 459, 511, 611) ou de l'autre élément est configurée pour recevoir en son sein la vis sans fin (314, 514, 614) et au moins une partie du moteur électrique (305, 405, 505, 605, 715).

8. Prothèse de doigt (120, 220, 300, 400, 700) selon l'une quelconque des revendications 5 à 7, dans laquelle l'actionneur (301, 404, 501, 601, 704) comprend en outre un palier de butée configuré pour retenir de manière axiale la vis sans fin (314, 514, 614).

9. Prothèse de doigt (120, 220, 300, 400, 700) selon l'une quelconque des revendications 5 à 8, dans laquelle une rotation de la vis sans fin (314, 514, 614) selon un premier sens de rotation amène le logement (311, 403, 459, 511, 611) ou l'autre élément à réaliser une translation de manière distale par rapport à la vis sans fin (314, 514, 614) et une rotation de la vis sans fin (314, 514, 614) selon un second sens de rotation opposé au premier sens de rotation amène le logement (311, 403, 459, 511, 611) ou l'autre élément à réaliser une translation de manière proximale par rapport à la vis sans fin (314, 514, 614).

10. Actionneur (301) selon l'une quelconque des revendications 1 à 4, dans lequel le logement (311) ou l'autre élément comprend une crémaillère (380).

11. Actionneur (301) selon la revendication 10, dans lequel la crémaillère (380) inclut une partie allongée présentant des filets partiels (386), et où les filets partiels (386) s'étendent transversalement le long de la longueur de la crémaillère (380).

12. Actionneur (301) selon l'une quelconque des revendications 10 et 11, dans lequel la crémaillère (380) comprend une jonction (388), la jonction (388) étant attachée sur le segment de doigt proximal (320).

13. Prothèse de doigt (300) selon l'une quelconque des revendications 5 à 9, dans laquelle le logement (311) ou l'autre élément comprend une crémaillère (380).

14. Prothèse de doigt (300) selon la revendication 13, dans laquelle la crémaillère (380) est une crémaillère à vis sans fin.

15. Prothèse de doigt (300) selon l'une quelconque des revendications 13 et 14, dans laquelle la crémaillère (380) inclut une partie allongée présentant des filets (386), et où les filets s'étendent transversalement le long de la longueur de la crémaillère (380).
